# EUROPEAN PATENT SPECIFICATION

(11) **EP 1 452 205 B1**
(45) Date of publication and mention of the grant of the patent: **28.11.2007**
(21) Application number: 04003545.3
(22) Date of filing: 15.10.1997
(51) Int. Cl.: A61K 38/18, A61P 17/02

(54) **Uses of keratinocyte growth factor-2**
Verwendung von keratinocyte growth factor-2
Utilisation du facteur de croissance des kératinocytes -2

(30) Priority: 15.10.1996 US 28495 P; 06.12.1996 US 32253 P; 10.12.1996 US 33457 P
(43) Date of publication of application: 01.09.2004
(62) Divisional of application: 97913676.9
(73) Proprietor: AMGEN INC., Thousand Oaks, CA 91320-1799 (US)
(72) Inventor: Lacey, David L., Thousand Oaks, CA 91320 (US); Ulich, Thomas R., Camarillo, CA 93012 (US); Danilenko, Dimitry M., Camarillo, CA 93012 (US); Farrell, Catherine L., Agoura, California 91301 (US)
(74) Representative: Grünecker, Kinkeldey, Stockmair & Schwanhäusser Anwaltssozietät

(56) References cited:
- EP-A- 0 619 370
- WO-A-96/25422
- WO-A-97/13857
- WO-A-98/16642
- RUBIN J S ET AL: "KERATINOCYTE GROWTH FACTOR" 1995, CELL BIOLOGY INTERNATIONAL, ACADEMIC PRESS, GB, PAGE(S) 399-411 , XP000909980 ISSN: 1065-6995 * the whole document *

## Description

### FIELD OF THE INVENTION

The present invention relates to the use of keratinocyte growth factor-2 (KGF-2) protein products to stimulate the proliferation, growth and differentiation of a variety of epithelial cells.

### BACKGROUND OF THE INVENTION

The complex process of tissue generation and regeneration is mediated by a number of protein factors sometimes referred to as soft tissue growth factors. These molecules are generally released by one cell type and act to influence proliferation of other cell types (Rubin et al. (1989), Proc. Nat'l. Acad. Sci. USA, 86:802-806). There are also some growth factors released from cells that themselves have the capacity to respond to such growth factors. Some soft tissue growth factors are secreted by particular cell types and influence the proliferation, differentiation, and/or maturation of responsive cells in the development of multicellular organisms (Finch et al. (1989), Science, 245:757-755). In addition to their roles in developing organisms, some soft tissue growth factors are significant in the continued health and maintenance of more mature systems. For instance, in mammals there are many systems where rapid cell turnover occurs. Such systems include the skin and the gastrointestinal tract, both of which are comprised of epithelial cells. Included within this group of soft tissue growth factors is a protein family of fibroblast growth factors (FGFs).

The fibroblast growth factor (FGF) family is now known to consist of at least fourteen members, namely FGF-1 to FGF-10 and homologous factors FHF-1 to FHF-4, which share a relatedness among primary structures: basic fibroblast growth factor, bFGF (Abraham et al. (1986), EMBO J., 5:2523-2528); acidic fibroblast growth factor, aFGF (Jaye et al. (1986), Science, 233:541-545); int-2 gene product, int-2 (Dickson & Peters (1987), Nature, 326:833); hst/kFGF (Delli-Bovi et al. (1987), Cell, 50:729-737 and Yoshida et al. (1987), Proc. Natl. Acad. Sci. USA, 84:7305-7309); FGF-5 (Zhan et al. (1988), Mol. Cell. Biol., 8:3487-3495); FGF-6 (Marics et al. (1989), Oncogene, 4:335-340); keratinocyte growth factor, KGF (Finch et al. (1989), Science, 24:752-755); hisactophilin (Habazzettl et al. (1992), Nature, 359:855-858); FGF-9 (Miyamoto et al. (1993), Mol. Cell Biol., 13(7):4251-4259); and fibroblast growth factor-10, also known as keratinocyte growth factor-2, KGF-2 (PCT patent application WO 96/25422), the disclosures of which are hereby incorporated by reference. More recently, four homologous factors (or "FHFs") were identified from the human retina by a combination of random cDNA sequencing, searches of existing sequence databases and homology-based polymerase chain reactions (Smallwood et al. (1996), Proc. Natl. Acad. Sci. USA, 93:9850-9857). It has been proposed that FHF-1, FHF-2, FHF-3 and FHF-4 should be designated as FGF-11, FGF-12, FGF-13 and FGF-14, respectively, in accordance with the recommendation of the Nomenclature Committee (Coulier et al. (1997), Journal of Molecular Evolution, 44:43-56).

WO 96/25422 describes the cloning, expression and purification of full-length (with signal sequence, residues Met¹ to Thr³⁶ of SEQ ID NO:2) and mature (without signal sequence, residues Cys³⁷ to Ser²⁰⁸ of SEQ ID NO:2) KGF-2 in a bacterial expression system (e.g., *E. coli*) and eukaryotic expression systems (e.g., baculovirus and COS cells). This reference further teaches that KGF-2 might be useful to stimulate cell growth and proliferation for new blood vessel growth or angiogenesis, the prevention of hair loss, the healing of dermal wounds and the differentiation of muscle cells, nervous tissue, prostate cells and lung cells.

Although KGF-2 is a member of the FGF family, the physical and *in vivo* effects among the family members is not the same. For example, it was well known that aFGF, bFGF and KGF respond to heparin in different ways. The mitogenic activity of aFGF is substantially increased in the presence of heparin, but the mitogenic activity of bFGF in the presence of heparin is only minimally increased, despite the fact that heparin tightly binds to bFGF. In contrast, thymidine incorporation by BALB/MK cells is inhibited when heparin is included with KGF in the culture medium (Ron et al. (1993), J. Biol. Chem., 268:2984-2988). Moreover, aFGF and bFGF are known to bind to the same receptor, but KGF also has different receptors on NIH/3T3 fibroblasts from the aFGF and bFGF receptors on NIH/3T3 fibroblasts, which fail to interact with KGF (Bottaro, et al. (1990), J. Biol. Chem., 265, 12767-12770). Additionally, KGF is distinct from aFGF and bFGF in that it is not mitogenic for fibroblasts or endothelial cells (Rubin et al. (1989), Proc. Natl. Acad. Sci. USA, 86:802-806).

In fact, the expression profile of KGF-2 is quite different from those of other members of the FGF family (Yamasaki et al. (1996), J. Biol Chem., 271:15918-15921). KGF-2 presumably has a unique physiological role, with its biological activity not having been elucidated (Emoto et al. (1997), J. Biol. Chem., 272(37):23191-23194).

Therefore, much remains to be learned regarding KGF-2 as a growth factor, including the epithelial cells in additional types of organs and tissues for which KGF-2 may be targeted and the *in vivo* effect of KGF-2 on such cells.

### SUMMARY OF THE INVENTION

This invention relates to the *in vivo* or *in vitro* use of KGF-2 protein product(s), as defined in the claims to stimulate the production of epithelial cells of the pancreas (exocrine and endocrine) liver and/or gastrointestinal tract including cells in the oral cavity, in the glandular stomach and small intestine, in the colon and in other cells within the intestinal mucosa.

### BRIEF DESCRIPTION OF THE FIGURES

Numerous aspects and advantages of the present invention will become apparent upon review of the Figures, wherein:
Figure 1 depicts a cDNA sequence (SEQ ID NO:1) encoding full-length, recombinant human KGF-2. Also depicted is the amino acid sequence (SEQ ID NO:2)- of full-length, recombinant human KGF-2. The initial 36 amino acid residues (Met¹ to Thr³⁶) represent the putative leader sequence of full-length KGF-2 and residues Cys³⁷ to Ser²⁰⁸ of SEQ ID NO:2 represent mature KGF-2. The full-length and mature forms are collectively termed "KGF-2".
Figure 2 depicts a cDNA sequence (SEQ ID NO:3) encoding His rFGF10. Also depicted is the amino acid sequence (SEQ ID NO:4) of His rFGF10.
Figure 3 depicts a cDNA sequence (SEQ ID NO:5) encoding hFGF10. Also depicted is the amino acid sequence (SEQ ID NO:6) of hFGF10.

### DETAILED DESCRIPTION OF THE INVENTION

In accordance with the present invention and as defined in the claims, KGF-2 protein product(s), as more fully described below, may be used *in vivo* or *in vitro* to stimulate production of epithelial cells of the pancreas (endocrine and exocrine) liver and gastrointestinal tract including cells in the oral cavity, in the glandular stomach and small intestine, in the colon and in other cells within the intestinal mucosa.

This invention thus has significant implications in terms of enabling the application of KGF-2 protein product(s) specifically characterized by the prophylactic and therapeutic use of KGF-2 to reduce, delay and/or block the onset of damage to or deficiencies in these particular types of cells. The following is a description of diseases and medical conditions which can be treated with KGF-2 protein product(s) in accordance with the invention.

KGF-2 protein product(s) are useful to increase cytoprotection, proliferation and/or differentiation of hepatocytes in order to increase liver function. KGF-2 protein product(s) are useful to treat and/or prevent hepatic cirrhosis, fulminant liver failure, damage caused by acute viral hepatitis, toxic insults to the liver and/or bile duct disorders.

Hepatic cirrhosis, secondary to viral hepatitis and chronic alcohol ingestion, is a significant cause of morbidity and mortality. KGF-2 protein product(s) are useful to treat and/or prevent the development of cirrhosis. A standard *in vivo* model of hepatic cirrhosis is known (Tomaszewski et al. (1991), J. Appl. Toxicol., 11:229-231).

Fulminant liver failure is a life-threatening condition which occurs with end-stage cirrhosis and which is presently treatable only with liver transplantation. KGF-2 protein product(s) are useful to treat and/or prevent fulminant liver failure. Standard *in vivo* models of fulminant liver failure are known (Mitchell et al. (1973), J. Pharmacol. Exp. Ther., 187:185-194; Thakore and Mehendale (1991), Toxicologic Pathol., 19:47-58; and Havill et al. (1994), FASEB Journal, 8(4-5):A930, Abstract 5387).

Acute viral hepatitis is frequently subclinical and self-limiting. However, in a minority of patients severe liver damage can result over several weeks. KGF-2 protein product(s) are useful to treat and/or prevent viral hepatitis. Standard *in vivo* models of hepatocyte proliferation are known (Housley et al. (1994), Journal of Clinical Investigation, 94(5):1764-1777; and Havill et al. (1994), *supra*).

Toxic insults to the liver caused by acetaminophen, halothane, carbon tetrachloride and other toxins may be prevented or treated by KGF-2 protein product(s). Standard *in vivo* models of liver toxicity are known (Mitchell et al. (1973), *supra*; Thakore and Mehendale (1991), *supra;* and Havill et al. (1994), *supra*).

KGF-2 protein product(s) are useful to increase cytoprotection, proliferation and/or differentiation of epithelial cells in the gastrointestinal tract (e.g., the oral cavity, esophagus, stomach, small intestine, colon, rectum and anal canal). The terms "gastrointestinal tract", as defined herein, and "gut" are art-recognized terms and are used interchangeably herein. Specifically, KGF-2 protein product(s) are useful to treat and/or prevent gastric ulcers, duodenal ulcers, inflammatory bowel disease, gut toxicity and erosions of the gastrointestinal tract.

Gastric ulcers cause significant morbidity, have a relatively high recurrence rate, and heal by scar formation on the mucosal lining. KGF-2 protein product(s) are useful to prevent degeneration of glandular mucosa and to regenerate glandular mucosa more rapidly, e.g., offering a significant therapeutic improvement in the treatment of gastric ulcers. Standard *in vivo* models of gastric ulcers are known (Tarnawski et al. (1991), "Indomethacin Impairs Quality of Experimental Gastric Ulcer Healing: A Quantitative Histological and Ultrastructural Analysis", In:Mechanisms of Injury, Protection and Repair of the Upper Gastrointestinal Tract, (eds) Garner and O'Brien, Wiley & Sons; Brodie (1968), Gastroenterology, 55:25; and Ohning et al. (1994), Gastroenterology, 106(4 Suppl.):A624).

Duodenal ulcers, like gastric ulcers, cause significant morbidity and have a relatively high recurrence rate. KGF-2 protein product(s) are useful to prevent degeneration of the mucosal lining of the duodenum and to rapidly regenerate the mucosal lining of the duodenum to heal those ulcers and decrease their recurrence. Standard *in vivo* models of duodenal ulcers are known (Berg et al. (1949), Proc. Soc. Exp. Biol. Med., 7:374-376; Szabo and Pihan, Chronobiol. Int. (1987), 6:31-42; and Robert et al. (1970), Gastroenterology, 59:95-102).

Gut toxicity is a major limiting factor associated with cancer treatment, both in radiation (abdominal, total body or local, e.g., head and neck) and chemotherapy. Of primary concern are those patients undergoing: chemotherapy for cancer such as leukemia, breast cancer or as an adjuvant to tumor removal; radiotherapy for head and neck cancer; and combined chemotherapy and radiotherapy for bone marrow transplants. The severity of damage is related to the type and dose of chemotherapeutic agent(s) and concomitant therapy such as radiotherapy.

Mucositis in portions of the gastrointestinal tract may account for significant pain and discomfort for these patients, and range in severity from redness and swelling to frank ulcerative lesions. The lesions often become secondarily infected and become much harder to heal. Standard *in vivo* models of radiation-induced gut toxicity are known (Withers and Elkind (1970), Int. J. Radiat., 17(3):261-267). Standard *in vivo* models of chemotherapy-induced gut toxicity are known (Farrell et al., The American Society of Hematology, 38th Annual Meeting (Orlando, FL), December 6-8, 1996; Sonis et al. (1990), Oral Surg. Oral Med & Oral Pathol., 69(4):437-443; and Moore (1985), Cancer Chemotherapy Pharmacol., 15:11-15).

Exemplary chemotherapeutic agents include, but are not limited to, BCNU, busulfan, carboplatin, cyclophosphamide, cisplatin, cytosine arabinoside, daunorubicin, doxorubicin, etoposide, 5-fluorouracil, gemcytabine, ifosphamide, irinotecan, melphalan, methotrexate, navelbine, topotecan, taxol and taxotere, and exemplary treatment regimes include, but are not limited to, BEAM (busulfan, etoposide, cytosine arabinoside, methotrexate); cyclophosphamide and total body irradiation; cyclophosphamide, total body irradiation and etoposide; cyclophosphamide and busulfan; and 5-fluorouracil with leucovorin or levamisole.

Treatment, pretreatment and/or post-treatment, with KGF-2 protein product(s) are useful to generate a cytoprotective effect or regeneration or both, for example, on the small intestinal mucosa, allowing increased dosages of such therapies while reducing potential fatal side effects of gut toxicity.

KGF-2 protein product(s) may preferentially be administered in the following settings. Colorectal patients routinely are administered 5-fluorouracil with leucovorin on days 1 to 5; KGF-2 protein product(s) may be administered on days -2, -1 and 0. Head and neck cancer patients routinely are administered hypofractionated radiation therapy, plus 5-fluorouracil and cisplatin over a seven week period; KGF-2 protein product(s) may be administered on days -2, -1 and 0 and thereafter once per week until the end of the radiation therapy. In lymphoma transplantation patients are frequently administered BEAM therapy for 6 days (days 1 to 6); KGF protein product (s) may be administered on days -2, -1 and 0 and as a three day post-treatment (days 7 to 9).

In specific embodiments, KGF-2 protein product(s) may be administered prophylactically and/or therapeutically to reduce, delay and/or block the onset of mucositis (due to chemotherapy and/or radiotherapy), in combination with one or more cytokines to delay and/or block the onset of cytopenia.

Typically, bone marrow, peripheral blood progenitor cells or stem cells (McNiece et al. (1989), Blood, 74:609-612 and Moore et al. (1979), Blood Cells, 5:297-311) are removed from a patient prior to myelosuppressive cytoreductive therapy (chemotherapy alone or with radiation therapy) and are then readministered to the patient concurrent with or following cytoreductive therapy in order to counteract the myelosuppressive effects of such therapy.

Many different approaches have been undertaken to protect an organism from the side effects of radiation or toxic chemicals. One approach is to replace bone marrow cells before toxicity has developed. Another approach is to use progenitor cells from the peripheral blood (PBPC). These PBPC can be collected by apheresis or phlebotomy following cytokine therapy alone (G-CSF or GM-CSF), or with chemotherapy or cytokines. They can be given back fresh or cryopreserved. If desired, the cells may be CD34+ selected, T-cell depleted, tumor cell depleted, or the progenitor cells can be expanded (caused to multiply) by means known in the art, prior to administration. The benefits of re-infusion of autologous or allogeneic progenitors following myelosuppressive therapy have been described in the literature (Morse et al. (1992), Ann. Clin. Lab. Sci., 22:221-225; Kessinger and Armitage (1991), Blood, 77:211-213; Kessinger et al. (1989), Blood, 74:1260-1265; Takam et al. (1989), Blood, 74:1245-1251 and Kessinger et al. (1988), Blood, 171:723-727).

As used herein, the term "cytokine" is a generic term for proteins released by one cell population which act on another cell as intercellular mediators. Examples of such cytokines are lymphokines, monokines and traditional polypeptide hormones. Included among the cytokines are insulin-like growth factors; human growth hormone; N-methionyl human growth hormone; bovine growth hormone; parathyroid hormone; thyroxine; insulin; proinsulin; relaxin; prorelaxin; glycoprotein hormones such as follicle stimulating hormone (FSH), thyroid stimulating hormone (TSH) and leutinizing hormone (LH); hemopoietic growth factor; hepatic growth factor; fibroblast growth factor; prolactin; placental lactogen; tumor necrosis factor alpha and -beta; mouse gonadotropin-associated peptide; inhibin; activin; vascular endothelial growth factor; integrin; thrombopoietin; nerve growth factors such as NGF-beta; platelet-growth factors such as TPO and MGDF; transforming growth factors (TGFs) such as TGF-alpha and TGF-beta; insulin-like growth factor-I and -II; erythropoietin; osteoinductive factors; interferons such as interferon-alpha, -beta and -gamma; colony stimulating factors (CSFs) such as macrophage-CSF (M-CSF), granulocyte-macrophage-CSF (GM-CSF), and granulocyte-CSF (G-CSF); interleukins (ILs) such as IL-1, IL-2, IL-3, IL-4, IL-5, IL-6, IL-7, IL-8, IL-9, IL-10, IL-11, IL-12, IL-13, IL-14, IL-15 and IL-16; and other polypeptide factors. The cytokines can be used alone or in combination to protect against, mitigate and/or reverse myeloid or hematopoietic toxicity associated with cytotoxic agents.

The administration of KGF-2 protein product(s), as well as of the cytokine, should be coordinated and optimized. Depending upon the circumstances, an appropriate dose of KGF-2 protein(s) can be administered prior to or subsequent to administration of the therapeutic agent(s). For example, a parameter to be considered is whether the cytokine is administered in a single dose or in multiple doses during the course of therapy. Certain cytokines are cleared rapidly from the body and will require periodic or continuous administration in order for their efficacy to be maximized. The manner of administration can differ, depending on whether a pre-treatment or post-treatment of the cytokine is given. For example, if the cytokine is given prior to the cytotoxic agent, it is preferable to administer the cytokine by intravenous bolus injection for several hours and, optionally, to repeat such administration on one or more days during and after completion of the cytotoxic therapy.

In a specific embodiment, KGF-2 protein product(s) are to be administered (e.g., intraveneously) at 0.1 to 500 micrograms/kg/dose, preferably up to about 200 micrograms/kg/dose, prior to (e.g., 1 to 3 days) and/or after chemotherapy or radiation therapy, and G-CSF (Neupogen^{™} or Lenograstim^{™}) or GM-CSF (Sargramostim^{™}) is to be administered (e.g., subcutaneously) at 5 micrograms/kg/dose for 1 to 10 days (preferably 7 to 10 days) after chemotherapy.

Erosions of the gastrointestinal tract (e.g., esophagus, stomach and intestine) include erosive gastritis, esophagitis, esophageal reflux and inflammatory bowel diseases. Inflammatory bowel diseases, such as Crohn's disease (affecting primarily the small intestine) and ulcerative colitis (affecting primarily the large bowel), are chronic diseases of unknown etiology which result in the destruction of the mucosal surface, inflammation, scar and adhesion formation during repair, and significant morbidity to the affected individuals. KGF-2 protein product(s) are useful to regenerate the mucosal lining and decrease the recurrence of these erosions, resulting in faster healing, and may be of benefit in controlling progression of the disease. Standard *in vivo* models of erosion of the gastrointestinal tract are known (Geisinger et al. (1990), Mod-Pathol., 3(5):619-629; Carlborg et al. (1983), Laryngoscope, 93(2):184-187; Carlborg et al. (1980), Eur-Surg-Res., 12(4):270-282; Keshavarzian et al. (1991), Alcohol-Clin-Exp-Res., 15(1):116-121; Katz et al. (1988), Dig-Dis-Sci., 33 (2) :217-224; and Zeeh et. al. (1996), Gastroenterology, 110(4):1077-1083). Standard *in vivo* models of inflammatory bowel disease are well known (Morris et al. (1989), Gastroenterology, 96:795-803; Rachmilewitz et al. (1989), Gastroenterology, 97:326-327; Allgayer et al. (1989), Gastroenterology, 96:1290-1300; and Kim and Borstad (1992), Scand. J. Gastroenterol, 27(7):529-537).

Animal studies have established the relationship between total parenteral nutrition (TPN) and intestinal mucosal atrophy (Buchman et al. (1995), Journal of Parenteral and Enteral Nutrition, 19:453-460). The decrease in intestinal villus height is attributed to the lack of growth stimulus provided through oral intake of nutrients. This is reflected in a reduction in the labeling index, a measure of growth. Decreases in villus height are also correlated with decreases in specific activities of enzymes involved in nutrient absorption. KGF-2 protein product(s) are useful to either protect against atrophy during the fasting and/or facilitate regrowth upon reintroduction of oral nutrients.

Disorders of the pancreas may be endocrine-related such as Type I or Type II diabetes, or may be exocrine-related such as pancreatitis and pancreatic inefficiencies or cystic fibrosis. Patients with diagnosed Type I diabetes require constant exogenous insulin administration. Patients with diagnosed Type II diabetes progress through varying stages of insulin resistance/insufficiency to ultimately also require exogenous insulin administration. KGF-2 protein product(s) are useful to ameliorate, delay and/or circumvent permanent manifestation of diabetes mellitus or as an adjunct in the setting of islet cell transplantation by inducing pancreatic beta cell function in order to normalize blood glucose levels during varying metabolic demands, yet avoid frequent or profound hypoglycemia. Standard models of diabetes are known (Junod et al. (1967), Proc. Soc. Exp. Bio. Med. 126(1):201-205; Rerup (1970), Pharm. Rev., 22:485-518; Rossini et al. (1977), P.N.A.S., 74:2485-2489; and Ar' Rajab and Ahren (1993), Pancreas, 8:50-57). A standard model of pancreatic cell proliferation is known (Yi et al. (1994), American Journal of Pathology, 145(1) :80-85).

### KGF-2 protein(s)

In accordance with the terms of this invention, by the term "KGF-2 protein(s)" is meant the protein defined by amino acids Cys³⁷ to ser²⁰⁸ of SEQ ID NO: 2 (mature KGF-2) and variant proteins thereof. The term "KGF-2 protein(s)" thus includes a protein in which one or more amino acid residues have been deleted from ("deletion variant(s)"), inserted into ("addition variant (s)"), and/or substituted for ("substitution variant (s)") residues within the amino acid sequence of SEQ ID NO:2 and which retains biological activity. Thus, while the descriptions below of protein modifications refer to mature KGF-2, it does not preclude additional modifications thereto.

The term "biological activity" as used herein means that a KGF-2 protein(s) possesses some but not necessarily all the same properties of (and not necessarily to the same degree as) mature KGF-2. The selection of the particular properties of interest depends upon the desired use of the desired KGF-2 protein (s).

It will be appreciated by those skilled in the art that many combinations of deletions, insertions, and substitutions can be made, provided that the final protein is biologically active. There are two principal variables in the construction of amino acid sequence variant(s): the location of the mutation site and the nature of the mutation. In designing variant(s),'the location of the mutation site and the nature of the mutation will depend on the biochemical characteristic(s) to be modified. Mutation sites can be modified individually or in series, e.g., by
(1) deleting the target amino acid residue,
(2) inserting amino acid residues adjacent to the located site or (3) substituting first with conservative amino acid choices and, depending upon the results achieved, then with more radical selections.

Amino acid sequence deletions generally range from about 40 amino acid residues, from about 30 amino acids, from about 20 amino acids, and typically from about 1 to 10 residues. Deletions within the amino acid sequence of mature KGF-2 may be made, for example, in regions of low homology with the sequences of other members of the FGF family. Deletions within the amino acid sequence of mature KGF-2 in areas of substantial homology with the sequences of other members of the FGF family will be more likely to significantly modify the biological activity. The number of total deletions and/or consecutive deletions preferably will be selected so as to preserve the tertiary structure of mature KGF-2 in the affected domain, e.g., cysteine crosslinking.

Amino acid sequence additions may include amino- and/or carboxyl-terminal fusions ranging in length from one residue to one hundred or more residues, as well as internal intrasequence insertions of single or multiple amino acid residues. Internal additions may range preferably from about 1 to 10 amino acid residues, more preferably from about 1 to 5 amino acid residues, and most preferably from about 1 to 3 amino acid residues. Additions within the amino acid sequence of mature KGF-2 may be made in regions of low homology with the sequences of other members of the FGF family. Additions within the amino acid sequence of mature KGF-2 in areas of substantial homology with the sequences of other members of the FGF family will be more likely to significantly modify the biological activity. Insertions or additions preferably include amino acid sequences derived from the sequences of other FGF family members.

An amino-terminus addition is contemplated to include the addition of a methionine (for example, as an artifact of the direct expression in bacterial recombinant cell culture) or an amino acid residue or sequence of mature KGF-2. A further example of an N-terminal addition includes the fusion of a signal sequence to the N-terminus of mature KGF-2 in order to facilitate the secretion of protein from recombinant host cells. Such signal sequences generally will be obtained from and thus be homologous to the intended host cell species. Included within the scope of this invention is the native signal sequence, for example, the native signal sequence of the protein defined by amino acids Met¹ to Thr³⁶ of SEQ ID NO:2 or a heterologous signal sequence. A heterologous signal sequence selected should be one that is recognized and processed (i.e., cleaved by a signal peptidase) by the host cell. For prokaryotic host cells that do not recognize and process the native signal sequence, the signal sequence may be substituted by a prokaryotic signal sequence selected, for example, from the group of the alkaline phosphatase, penicillinase or heat-stable enterotoxin II leaders. For yeast secretion, the signal sequence may be selected, for example, from the group of the yeast invertase, alpha factor or acid phosphatase leader sequences. In mammalian cell expression specifically, signal sequences of full-length KGF-2 or of other FGF family members (e.g., KGF) may be suitable.

An example of a carboxy-terminus addition includes chimeric proteins comprising the fusion of KGF-2 with all or part of the constant domain of the heavy or light chain of human immunoglobulin. Such chimeric polypeptides are preferred wherein the immunoglobulin portion comprises all domains except the first domain of the constant region of the heavy chain of human immunoglobulin such as IgG, IgA, IgM or IgE, especially IgG, e.g., IgG1 or IgG3. A skilled artisan will appreciate that any amino acid of the immunoglobulin portion can be deleted or substituted by one or more amino acids, or one or more amino acids can be added as long as the KGF-2 portion still stimulates epithelial cells and the immunoglobulin portion shows one or more of its characteristic properties.

Another group of variant(s) is amino acid substitution variant(s) of the amino acid sequence of mature KGF-2. These variant(s) have at least one amino acid residue in the sequence of Cys³⁷ to Ser²⁰⁸ of SEQ ID NO:2 removed and a different residue inserted in its place. Substitution variant(s) include allelic variant(s), which are characterized by naturally-occurring nucleotide sequence changes in the species population that may or may not result in an amino acid change. One skilled in the art can use any information known about the binding or active site of the polypeptide in the selection of possible mutation sites.

One method for identifying amino acid residues or regions for mutagenesis is called "alanine scanning mutagenesis", as described by Cunningham and Wells (1989), *Science,* 244:1081-1085. In this method, an amino acid residue or group of target residues is identified (e.g., charged residues such as Arg, Asp, His, Lys, and Glu) and replaced by a neutral or negatively-charged amino acid (most preferably alanine or polyalanine) to effect the interaction of the amino acids with the surrounding aqueous environment in or outside the cell. Those domains demonstrating functional sensitivity to the substitutions are then refined by introducing additional or alternate residues at the sites of substitution. Thus, the site for introducing an amino acid sequence modification is predetermined and, to optimize the performance of a mutation at a given site, alanine scanning or random mutagenesis may be conducted and the variant (s) may be screened for the optimal combination of desired activity and degree of activity.

The sites of greatest interest for substitutional mutagenesis include sites in which particular residues within amino acids Cys³⁷ to Ser²⁰⁸ of SEQ ID NO:2 are substantially different from various species or other FGF family members in terms of side-chain bulk, charge, and/or hydrophobicity. Other sites of interest include those in which particular residues within amino acids Cys³⁷ to Ser²⁰⁸ of SEQ ID NO:2, are identical among various species or other FGF family members. Such positions are generally important for the biological activity of a protein. Accordingly, a skilled artisan would appreciate that initially these sites should be modified by substitution in a relatively conservative manner.

Such conservative substitutions are shown in Table 1 under the heading of "Preferred Substitutions". If such substitutions result in a change in biological activity, then more substantial changes (Exemplary Substitutions) may be introduced and/or other additions/deletions may be made and the resulting products screened.

**TABLE 1: Amino Acid Substitutions**

| Original Residue | Preferred Substitutions | Exemplary Substitutions |
|---|---|---|
| Ala (A) | Val | Val; Leu; Ile |
| Arg (R) | Lys | Lys; Gln; Asn |
| Asn (N) | Gln | Gln: His; Lys; Arg |
| Asp (D) | Glu | Glu |
| Cys (C) | Ser | Ser |
| Gln (Q) | Asn | Asn |
| Glu (E) | Asp | Asp |
| Gly (G) | Pro | Pro |
| His (H) | Arg | Asn; Gln; Lys; Arg |
| Ile (I) | Leu | Leu; Val; Met; Ala; Phe; norleucine norleucine; |
| Leu (L) | Ile | Ile; Val; Met; Ala; Phe |
| Lys (K) | Arg | Arg; Gln; Asn |
| Met (M) | Leu | Leu; Phe; Ile |
| Phe (F) | Leu | Leu; Val; Ile; Ala |
| Pro (P) | Gly | Gly |
| Ser (S) | Thr | Thr |
| Thr (T) | Ser | Ser |
| Trp (W) | Tyr | Tyr |
| Tyr (Y) | Phe | Trp; Phe; Thr; Ser |
| Val (V) | Leu | Ile; Leu; Met; Phe; Ala; norleucine |

In making such changes of an equivalent nature, the hydropathic index of amino acids may be considered. The importance of the hydropathic amino acid index in conferring interactive biological function on a protein is generally understood in the art (Kyte and Doolittle (1982), J. Mol. Biol., 157:105-131). It is known that certain amino acids may be substituted for other amino acids having a similar hydropathic index or score and still retain a similar biological activity.

It is also understood in the art that the substitution of like amino acids can be made effectively on the basis of hydrophilicity, particularly where the biological functional equivalent protein or peptide thereby created is intended for use in immunological embodiments, as in the present case. U.S. Patent 4,554,101, the disclosure of which is incorporated herein by reference, states that the greatest local average hydrophilicity of a protein, as governed by the hydrophilicity of its adjacent amino acids, correlates with its immunogenicity and antigenicity, i.e., with a biological property of the protein.

U.S. Patent 4,554,101 also teaches the identification and preparation of epitopes from primary amino acid sequences on the basis of hydrophilicity. Through the methods disclosed in U.S. Patent 4,554,101 a skilled artisan would be able to identify epitopes, for example, within the amino acid sequence of KGF-2. These regions are also referred to as "epitopic core regions". Numerous scientific publications have been devoted to the prediction of secondary structure, and to the identification of epitopes, from analyses of amino acid sequences (Chou and Fasman (1974), Biochemistry, 13(2):222-245; Chou and Fasman (1974), Biochemistry, 13(2):211-222; Chou and Fasman (1978), Adv. Enzymol. Relat. Areas Mol. Biol., 47:45-148; Chou and Fasman (1978), Ann. Rev. Biochem., 47:251-276 and Chou and Fasman (1979), Biophys. J., 26:367-384). Moreover, computer programs are currently available to assist with predicting antigenic portions and epitopic core regions of proteins. Examples include those programs based upon the Jameson-Wolf analysis (Jameson and Wolf (1988), Comput. Appl. Biosci., 4(1):181-186 and Wolf et al. (1988), Comput. Appl. Biosci., 4(1):187-191, the disclosures of which are incorporated herein by reference); the program PepPlot^{®} (Brutlag et al. (1990), CABS, 6:237-245 and Weinberger et al. (1985), Science, 228: 740-742); and other new programs for protein tertiary structure prediction (Fetrow and Bryant (1993), BIOTECHNOLOGY, 11:479-4-83).

In contrast, substantial modifications in the functional and/or chemical characteristics of the amino acids Cys³⁷ to Ser²⁰⁸ of SEQ ID NO:2 may be accomplished by selecting substitutions that differ significantly in their effect on maintaining (a) the structure of the polypeptide backbone in the area of the substitution, for example, as a sheet or helical conformation, (b) the relative charge or hydrophobicity of the protein at the target site or (c) the bulk of the side chain. Naturally-occurring residues are divided into groups based on common side chain properties:
1) hydrophobic: norleucine, Met, Ala, Val, Leu, Ile;
2) neutral hydrophilic: Cys, Ser, Thr;
3) acidic: Asp, Glu;
4) basic: Asn, Gln, His, Lys, Arg;
5) residues that influence chain orientation: Gly, Pro; and
6) aromatic: Trp, Tyr, Phe.

Non-conservative substitutions may involve the exchange of a member of one of these groups for another. Such substituted residues may be introduced into regions of amino acids Cys³⁷ to Ser²⁰⁸ of SEQ ID NO:2 that, for example, are homologous with regions of other FGF family members or into the non-homologous regions of the protein.

In a specific embodiment, a variant polypeptide will preferably be substantially homologous to amino acids Cys³⁷ to Ser²⁰⁸ of SEQ ID NO:2. The term "substantially homologous", as used herein, means having a degree of homology (i.e., identity of amino acid residues; to amino acids Cys³⁷ to Ser²⁰⁸ of SEQ ID NO:2 in excess of eighty percent (80%); preferably, in excess of ninety percent (90%); more preferably, in excess of ninety-five percent (95%); and most preferably, in excess of ninety-nine percent (99%). The percentage of homology as described herein is calculated as the percentage of amino acid residues found in the smaller of the two sequences which align with identical amino acid residues in the sequence being compared when four gaps in a length of 100 amino acids may be introduced to assist in that alignment, as set forth by Dayhoff (1972), in Atlas of Protein Sequence and Structure, 5:124, National Biochemical Research Foundation, Washington, D.C., the disclosure of which is hereby incorporated by reference. Also included as substantially homologous are variant(s) of the amino acids Cys³⁷ to Ser²⁰⁸ of SEQ ID NO:2 which may be isolated by virtue of cross-reactivity with antibodies to amino acids Cys³⁷ to Ser²⁰⁸ of SEQ ID NO: 2, or whose genes may be isolated through hybridization with the DNA of SEQ ID NO:1 or with segments thereof.

It will be appreciated by those skilled in the art that many combinations of deletions, insertions and substitutions can be made, provided that the final KGF-2 protein(s) are biologically active. A KGF-2 protein(s) may be rapidly screened to assess its physical properties. For example, the level of biological activity (e.g., receptor binding and/or affinity, mitogenic, cell proliferative and/or *in vivo* activity) may be tested using a variety of assays. One such assay includes a mitogenic assay to test the ability of a protein to stimulate DNA synthesis (Rubin et al. (1989), *supra*). Another such assay includes a cell proliferative assay to test the ability of a protein to stimulate cell proliferation (Falco et al. (1988), Oncogene, 2:573-578).

### Polypeptide Derivatives

Chemically modified derivatives of KGF-2 protein(s) in which the polypeptide is linked to a polymer in order to modify properties (referred to herein as "derivatives"), are included within the scope of the present invention. Chemically modified derivatives of KGF-2 protein(s) may be prepared by one skilled in the art given the disclosures herein. Conjugates may be prepared using glycosylated, non-glycosylated or de-glycosylated KGF-2 protein(s). Typically, non-glycosylated KGF-2 protein(s) will be used.

Suitable chemical moieties for derivatization include water soluble polymers. Water soluble polymers are desirable because the protein to which each is attached will not precipitate in an aqueous environment, such as a physiological environment. Preferably, the polymer will be pharmaceutically acceptable for the preparation of a therapeutic product or composition. One skilled in the art will be able to select the desired polymer based on such considerations as whether the polymer/protein conjugate will be used therapeutically and, if so, the therapeutic profile (e.g., the duration of sustained release; resistance to proteolysis, the effects, if any, on dosage, biological activity; the ease in handling; the degree or lack of antigenicity and other known effects of a water soluble polymer on a therapeutic protein).

Suitable, clinically acceptable, water soluble polymers include, but are not limited to, polyethylene glycol (PEG), polyethylene glycol propionaldehyde, copolymers of ethylene glycol/propylene glycol, monomethoxy-polyethylene glycol, carboxymethylcellulose, dextran, polyvinyl alcohol (PVA), polyvinyl pyrrolidone, poly-1, 3-dioxolane, poly-1,3,6-trioxane, ethylene/maleic anhydride copolymer, poly (β-amino acids) (either homopolymers or random copolymers), poly(n-vinyl pyrrolidone)polyethylene glycol, polypropylene glycol homopolymers (PPG) and other polyalkylene oxides, polypropylene oxide/ethylene oxide copolymers, polyoxyethylated polyols (POG) (e.g., glycerol) and other polyoxyethylated polyols, polyoxyethylated sorbitol, or polyoxyethylated glucose, colonic acids or other carbohydrate polymers, Ficoll or dextran and mixtures thereof. As used herein, polyethylene glycol is meant to encompass any of the forms that have been used to derivatize other proteins, such as mono-(C1-C10) alkoxy- or arylbxy-polyethylene glycol. Polyethylene glycol propionaldehyde may have advantages in manufacturing due to its stability in water.

The water soluble polymers each may be of any molecular weight and may be branched or unbranched. Generally, the higher the molecular weight or the more branches, the higher the polymer:protein ratio. The water soluble polymers each typically have an average molecular weight of between about 2kDa to about 100kDa (the term "about" indicating that in preparations of a water soluble polymer, some molecules will weigh more, some less, than the stated molecular weight). The average molecular weight of each water soluble polymer preferably is between about 5kDa and about 40kDa, more preferably between about 10kDa and about 35kDa and most preferably between about 15kDa and about 30kDa.

There are a number of attachment methods available to those skilled in the art, including acylation reactions or alkylation reactions (preferably to generate an N-terminal chemically modified protein) with a reactive water soluble molecule. See, for example, EP 0 401 384, the disclosure of which is hereby incorporated by reference; see also, Malik et al. (1992), Exp. Hematol., 20.:1028-1035; Francis (1992), Focus on Growth Factors, 3(2) :4-10, published by Mediscript, Mountain Court, Friern Barnet Lane, London N20 OLD, UK; EP 0 154 316; EP 0 401 384; WO 92/16221; WO 95/34326; WO 95/13312; WO 96/11953; PCT International Application No. US96/19459; and the other publications cited herein that relate to pegylation.

A specific embodiment of the present invention is an unbranched monomethoxy-polyethylene glycol aldehyde molecule having an average molecular weight of about 20kDa conjugated via reductive alkylation to the N-terminus of a KGF-2 protein(s).

### Polyvalent Forms

Polyvalent forms, i.e., molecules comprising more than one active moiety, may be constructed. In one embodiment, the molecule may possess multiple KGF-2 protein(s). Additionally, the molecule may possess at least one KGF-2 protein(s) and, depending upon the desired characteristic of polyvalent form, at least one other molecule.

In one embodiment, KGF-2 protein(s) may be chemically coupled. For example, KGF-2 protein(s) may be chemically coupled to a divalent water soluble polymer via the pegylation technology described above. Additionally, KGF-2 protein(s) may be chemically coupled to a to biotin, and the biotin/KGF-2 protein(s) which are conjugated are then allowed to bind to avidin, resulting in tetravalent avidin/biotin/KGF-2 protein(s). KGF-2 protein(s) may also be covalently coupled to dinitrophenol (DNP) or trinitrophenol (TNP) and the resulting conjugates precipitated with anti-DNP or anti-TNP-IgM to form decameric conjugates.

In yet another embodiment, a recombinant fusion protein may also be produced having a KGF-2 protein(s) wherein each recombinant chimeric molecule has a KGF-2 protein(s) sequence, as described above, substituted for the variable domains of either or both of the immunoglobulin molecule heavy and light chains and having all or parts of the constant domains, but at least one constant domain, of the heavy or light chain of human immunoglobulin. For example, each such chimeric KGF-2 protein(s)/IgG1 fusion protein may be produced from two chimeric genes: a KGF-2 protein(s)/human kappa light chain chimera (KGF-2 protein(s)/Ck) and a KGF-2 protein(s)/human gamma-1 heavy chain chimera (KGF-2 protein(s)/Cg-1). Following transcription and translation of the two chimeric genes, as described below, the gene products may be assembled into a single chimeric molecule having a KGF-2 protein(s) displayed bivalently. Additional details relating to the construction of such chimeric molecules are disclosed in United States Patent 5,116,964, PCT Publication No. WO 89/09622, PCT Publication No. WO 91/16437 and EP 315062.

In yet a further embodiment, recombinant fusion proteins may also be produced wherein each recombinant chimeric molecule has at least one KGF-2 protein(s), as described above, and at least a portion of the region 186-401 of osteoprotegerin (OPG), as described in European Patent Application No. 96309363.8.

The production of KGF-2 protein(s) is described in further detail below. Such proteins may be prepared, for example, by recombinant techniques or by *in vitro* chemical synthesis of the desired KGF-2 protein (s) .

### Polynucleotides

Based upon the present description and using the universal codon table, one of ordinary skill in the art can readily determine all of the nucleic acid sequence which encodes an amino acid sequence of a KGF-2 protein(s).

Recombinant expression techniques conducted in accordance with the descriptions set forth below may be followed to produce these polynucleotides and to express the encoded proteins. For example, by inserting a nucleic acid sequence which encodes a KGF-2 protein(s) into an appropriate vector, one skilled in the art can readily produce large quantities of the desired nucleotide sequence. The sequences can then be used to generate detection probes or amplification primers. Alternatively, a polynucleotide encoding a KGF-2 protein(s) can be inserted into an expression vector. By introducing the expression vector into an appropriate host, the desired KGF-2 protein(s) may be produced in large amounts.

As further described herein, there are numerous host/vector systems available for the propagation of desired nucleic acid sequences and/or the production of KGF-2 protein(s). These include, but are not limited to, plasmid, viral and insertional vectors, and prokaryotic and eukaryotic hosts. One skilled in the art can adapt a host/vector system which is capable of propagating or expressing heterologous DNA to produce or express the sequences of the present invention.

Furthermore, it will be appreciated by those skilled in the art that, in view of the present disclosure, the nucleic acid sequences include the nucleic acids 109 to 624 of SEQ ID NO:1, as well as degenerate nucleic acid sequences thereof, nucleic acid sequences which encode variant(s) of mature KGF-2 and those nucleic acid sequences which hybridize (under hybridization conditions disclosed in the cDNA library screening section below, or equivalent conditions or more stringent conditions) to complements of nucleic acids 109 to 624 of SEQ ID NO:1.

Also provided by the present invention are recombinant DNA constructs involving vector DNA together with the DNA sequences encoding KGF-2 protein(s). In each such DNA construct, the nucleic acid sequence encoding a KGF-2 protein(s) (with or Without signal peptides) is in operative association with a suitable expression control or regulatory sequence capable of directing the replication and/or expression of the KGF-2 protein(s) in a selected host.

### Preparation of Polynucleotides

A nucleic acid sequence encoding a KGF-2 protein(s) can readily be obtained in a variety of ways including, without limitation, chemical synthesis, cDNA or genomic library screening, expression library screening, and/or PCR amplification of cDNA. These methods and others which are useful for isolating such nucleic acid sequences are set forth in Sambrook et al. (1989), Molecular Cloning: A Laboratory Manual, Cold Spring Harbor Laboratory Press, Cold Spring Harbor, NY; by Ausubel et al. (1994), eds Current Protocols in Molecular Biology, Current Protocols Press; and by Berger and Kimmel (1987), Methods in Enzymology: Guide to Molecular Cloning Techniques, Vol. 152, Academic Press, Inc., San Diego, CA.

Chemical synthesis of nucleic acid sequences which encode desired proteins can be accomplished using methods well known in the art, such as those set forth by Engels et al. (1989), Angew. Chem. Intl. Ed., 28:716-734 and Wells et al. (1985), Gene, 34:315. These methods include, *inter alia,* the phosphotriester, phosphoramidite and H-phosphonate methods of nucleic acid sequence synthesis. Large nucleic acid sequences, for example those larger than about 100 nucleotides in length, can be synthesized as several fragments. The fragments can then be ligated together to form a suitable nucleic acid sequence. A preferred method is polymer-supported synthesis using standard phosphoramidite chemistry.

Alternatively, a suitable nucleic acid sequence may be obtained by screening an appropriate cDNA library (i.e., a library prepared from one or more tissue sources believed to express the protein) or a genomic library (a library prepared from total genomic DNA). The source of the cDNA library is typically a tissue from any species that is believed to express a desired protein in reasonable quantities. The source of the genomic library may be any tissue or tissues from any mammalian or other species believed to harbor a gene encoding a KGF-2 protein(s).

Hybridization mediums can be screened for the presence of a DNA encoding a KGF-2 protein(s) using one or more nucleic acid probes (oligonucleotides, cDNA or genomic DNA fragments that possess an acceptable level of homology to the cDNA or gene to be cloned) that will hybridize selectively with cDNA(s) or gene(s) present in the library. The probes typically used for such screening encode a small region of DNA sequence from the same or a similar species as the species from which the library is prepared. Alternatively, the probes may be degenerate, as discussed herein.

Hybridization is typically accomplished by annealing the oligonucleotide probe or cDNA to the clones under conditions of stringency that prevent non-specific binding but permit binding of those clones that have a significant level of homology with the probe or primer. Typical hybridization and washing stringency conditions depend in part on the size i.e., number of nucleotides in length) of the cDNA or oligonucleotide probe and whether the probe is degenerate. The probability of identifying a clone is also considered in designing the hybridization medium (e.g., whether a cDNA or genomic library is being screened).

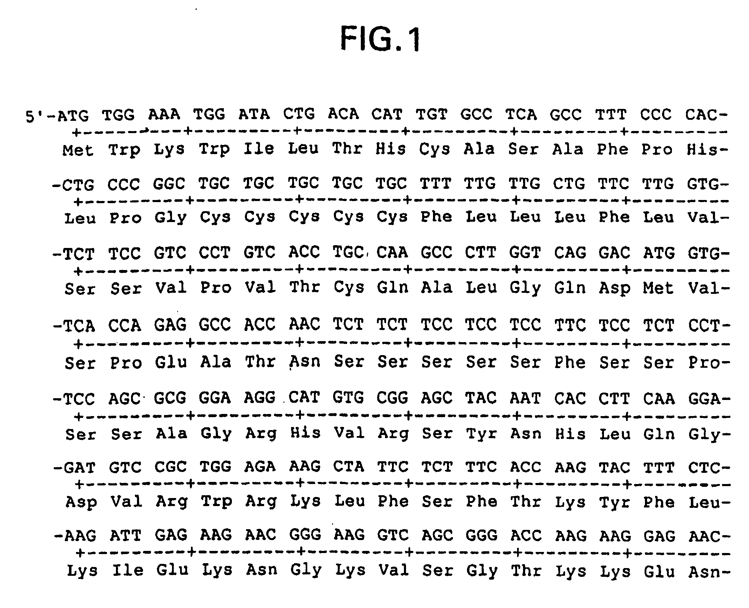

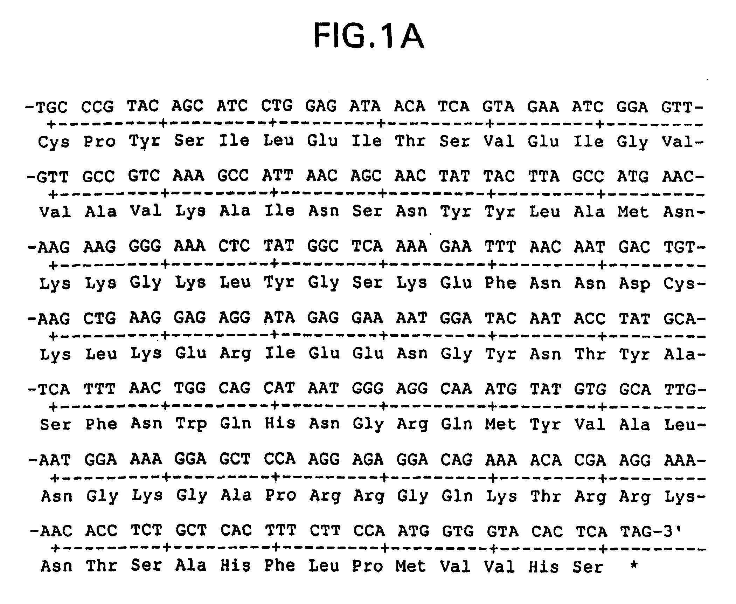

Where a DNA fragment (such as cDNA) is used as a probe, typical hybridization conditions include those as set forth in Ausubel et al. (1994), *eds., supra.* After hybridization, the hybridization medium is washed at a suitable stringency, depending on several factors such as probe size, expected homology of probe to clone, the hybridization medium being screened, the number of clones being screened, and the like. Exemplary stringent hybridization conditions are hybridization in 4 x SSC at 62-67°C, followed by washing in 0.1 x SSC at 62-67°C for approximately an hour. Alternatively, exemplary stringent hybridization conditions are hybridization in 45-55% formamide, 4 x SSC at 40-45°C. Also included are DNA sequences which hybridize to the nucleic acid sequences set forth in Figure 1 under relaxed hybridization conditions and which encode KGF-2 protein(s). Examples of such relaxed stringency hybridization conditions are 4 x SSC at 45-55°C or hybridization with 30-40% formamide at 40-45°C. See Maniatis et al. (1982), Molecular Cloning (A Laboratory Manual), Cold Spring Harbor Laboratory, pages 387 to 389.

There are also exemplary protocols for stringent washing conditions where oligonucleotide probes are used to screen hybridization mediums. For example, a first protocol uses 6 X SSC with 0.05 percent sodium pyrophosphate at a temperature of between about 35°C and 63°C, depending on the length of the probe. For example, 14 base probes are washed at 35-40°C, 17 base probes at 45-50°C, 20 base probes at 52-57°C, and 23 base probes at 57-63°C. The temperature can be increased 2-3°C where the background non-specific binding appears high. A second protocol uses tetramethylammonium chloride (TMAC) for washing. One such stringent washing solution is 3 M TMAC, 50mM Tris-HCl, pH 8.0 and 0.2% SDS.

Another method for obtaining a suitable nucleic acid sequence is the polymerase chain reaction (PCR). In this method, cDNA is prepared from poly(A)+RNA or total RNA using the enzyme reverse transcriptase. Two primers, typically complementary to two separate regions of cDNA (oligonucleotides) encoding a KGF-2 protein(s), are then added to the cDNA along with a polymerase such as Taq polymerase, and the polymerase amplifies the cDNA region between the two primers.

The oligonucleotide sequences selected as probes or primers should be of adequate length and sufficiently unambiguous so as to minimize the amount of non-specific binding that may occur during screening or PCR amplification. The actual sequence of the probes or primers is usually based on conserved or highly homologous sequences or regions. Optionally, the probes or primers can be fully or partially degenerate, i.e., can contain a mixture of probes/primers, all encoding the same amino acid sequence but using different codons to do so. An alternative to preparing degenerate probes is to place an inosine in some or all of those codon positions that vary by species. The oligonucleotide probes or primers may be prepared by chemical synthesis methods for DNA, as described above.

### Vectors

DNA encoding a KGF-2 protein(s) may be inserted into vectors for further cloning (amplification of the DNA) or for expression. Suitable vectors are commercially available, or the vector may be specifically constructed. The selection or construction of an appropriate vector will depend on (1) whether it is to be used for DNA amplification or for DNA expression, (2) the size of the DNA to be inserted into the vector, and (3) the intended host cell to be transformed with the vector.

The vectors each involve a nucleic acid sequence which encodes a desired protein operatively linked to one or more of the following expression control or regulatory sequences capable of directing, controlling or otherwise affecting the expression of a desired protein by a selected host cell. Each vector contains various components, depending on its function (amplification of DNA or expression of DNA) and its compatibility with the intended host cell. The vector components generally include, but are not limited to, one or more of the following: a signal sequence, an origin of replication, one or more selection or marker genes, promoters, enhancer elements, a transcription termination sequence and the like. These components may be obtained from natural sources or be synthesized by known procedures.

Examples of suitable prokaryotic cloning vectors include bacteriophages, such as lambda derivatives, or plasmids from *E. coli* (e.g. pBR322, col E1, pUC, the F-factor and Bluescript^{®} plasmid derivatives (Stratagene, LaJolla, CA)). Other appropriate expression vectors, of which numerous types are known in the art for the host cells described below, can also be used for this purpose.

### Signal Sequence

The nucleic acid encoding a signal sequence may be inserted 5' of the sequence encoding a KGF-2 protein(s), e.g, it may be a component of a vector or it may be a part of a nucleic acid encoding a KGF-2 protein(s). The nucleic acid encoding the native signal sequence of KGF-2 is known (WO 96/25422).

### Origin of Replication

Expression and cloning vectors each generally include a nucleic acid sequence that enables the vector to replicate in one or more selected host cells. In a cloning vector, this sequence is typically one that enables the vector to replicate independently of the host chromosomal DNA and includes origins of replication or autonomously replicating sequences. Such sequences are well known. The origin of replication from the plasmid pBR322 is suitable for most Gram-negative bacteria, and various origins (e.g., SV40, polyoma, adenovirus, VSV or BPV) are useful for cloning vectors in mammalian cells. Generally, the origin of replication is not needed for mammalian expression vectors (for example, the SV40 origin is often used only because it contains the early promoter).

### Selection Gene

The expression and cloning vectors each typically contain a selection gene. This gene encodes a "marker" protein necessary for the survival or growth of the transformed host cells when grown in a selective culture medium. Host cells that are not transformed with the vector will not contain the selection gene and, therefore, they will not survive in the culture medium. Typical selection genes encode proteins that (a) confer resistance to antibiotics or other toxins, e.g., ampicillin, neomycin, methotrexate or tetracycline; (b) complement auxotrophic deficiencies; or (c) supply critical nutrients not available from the culture medium.

Other selection genes may be used to amplify the genes to be expressed. Amplification is the process wherein genes which are in greater demand for the production of a protein critical for growth are reiterated in tandem within the chromosomes of successive generations of recombinant cells. Examples of suitable selectable markers for mammalian cells include dihydrofolate reductase (DHFR) and thymidine kinase. The cell transformants are placed under selection pressure which only the transformants are uniquely adapted to survive by virtue of the marker present in the vector. Selection pressure is imposed by culturing the transformed cells under conditions in which the concentration of selection agent in the medium is successively changed, thereby leading to amplification of both the selection gene and the DNA that encodes the desired protein. As a result, increased quantities of the desired protein are synthesized from the amplified DNA.

For example, cells transformed with the DHFR selection gene are first identified by culturing all of the transformants in a culture medium that contains methotrexate, a competitive antagonist of DHFR. An appropriate host cell when wild-type DHFR is used is the Chinese hamster ovary cell line deficient in DHFR activity (Urlaub and Chasin (1980), Proc. Natl. Acad. Sci., USA, 77(7):4216-4220). The transformed cells are then exposed to increased levels of methotrexate. This leads to the synthesis of multiple copies of the DHFR gene and, concomitantly, multiple copies of other DNA present in the expression vector, such as the DNA encoding the desired protein.

### Promoter

Expression and cloning vectors each will typically contain a promoter that is recognized by the host organism and is operably linked to a nucleic acid sequence encoding the desired protein. A promoter is an untranslated sequence located upstream (5') to the start codon of a structural gene (generally within about 100 to 1000 bp) that controls the transcription and translation of a particular nucleic acid sequence. A promoter may be conventionally grouped into one of two classes, inducible promoters and constitutive promoters. An inducible promoter initiates increased levels of transcription from DNA under its control in response to some change in culture conditions, such as the presence or absence of a nutrient or a change in temperature. A large number of promoters, recognized by a variety of potential host cells, are well known. A promoter may be operably linked to DNA encoding the desired protein by removing the promoter from the source DNA by restriction enzyme digestion and inserting the desired promoter sequence. The native KGF-2 promoter sequence may be used to direct amplification and/or expression of DNA encoding a desired protein. A heterologous promoter is preferred, however, if it permits greater transcription and higher yields of the expressed protein as compared to the native promoter and if it is compatible with the host cell system that has been selected for use. For example, any one of the native promoter sequences of other FGF family members may be used to direct amplification and/or expression of the DNA encoding a desired protein.

Promoters suitable for use with prokaryotic hosts include the beta-lactamase and lactose promoter systems; alkaline phosphatase, a tryptophan (trp) promoter system; a bacterial luminescence (luxR) gene system; and hybrid promoters such as the tac promoter. Other known bacterial promoters are also suitable. Their nucleotide sequences have been published, thereby enabling one skilled in the art to ligate them to the desired DNA sequence(s) using linkers or adaptors as needed to supply any required restriction sites.

Suitable promoting sequences for use with yeast hosts are also well known in the art. Suitable promoters for use with mammalian host cells are well known and include those obtained from the genomes of viruses such as polyoma virus, fowlpox virus, adenovirus (such as Adenovirus 2), bovine papilloma virus, avian sarcoma virus, cytomegalovirus, a retrovirus, hepatitis-B virus and, most preferably, Simian Virus 40 (SV40). Other suitable mammalian promoters include heterologous mammalian promoters, e.g., heat-shock promoters and the actin promoter.

### Enhancer Element

The expression and cloning vectors each will typically contain an enhancer sequence to increase the transcription by higher eukaryotes of a DNA sequence encoding a desired protein. Enhancers are cis-acting elements of DNA, usually from about 10-300 bp in length, that act on the promoter to increase its transcription.

Enhancers are relatively orientation and position independent. They have been found 5' and 3' to the transcription unit. Yeast enhancers are advantageously used with yeast promoters. Several enhancer sequences available from mammalian genes are known (e.g., globin, elastase, albumin, alpha-feto-protein and insulin). Additionally, viral enhancers such as the SV40 enhancer, the cytomegalovirus early promoter enhancer, the polyoma enhancer and adenovirus enhancers are exemplary enhancing elements for the activation of eukaryotic promoters. While an enhancer may be spliced into a vector at a position 5' or 3' to a DNA encoding a desired protein, it is typically located at a site 5' from the promoter.

### Transcription Termination

Expression vectors used in eukaryotic host cells each will typically contain a sequence necessary for the termination of transcription and for stabilizing the mRNA. Such sequences are commonly available from the 5' and occasionally 3' untranslated regions of eukaryotic DNAs or cDNAs. These regions contain nucleotide segments transcribed as polyadenylated fragments in the untranslated portion of the mRNA encoding a desired protein.

The construction of a suitable vector containing one or more of the above-listed components (together with the desired coding sequence) is accomplished by standard ligation techniques. Isolated plasmids or DNA fragments are cleaved, tailored and religated in the desired order to generate the required vector. To confirm that the correct sequence has been constructed, the ligation mixture may be used to transform *E. coli*, and successful transformants may be selected by known techniques as described above. Quantities of the vector from the transformants are then prepared, analyzed by restriction endonuclease digestion, and/or sequenced to confirm the presence of the desired construct.

A vector that provides for the transient expression of DNA encoding a desired protein in mammalian cells may also be used. In general, transient expression involves the use of an expression vector that is able to replicate efficiently in a host cell, such that the host cell accumulates many copies of the expression vector and, in turn, synthesizes high levels of the desired protein encoded by the expression vector. Each transient expression system, comprising a suitable expression vector and a host cell, allows for the convenient positive identification of proteins encoded by cloned DNAs, as well as for the rapid screening of such proteins for desired biological or physiological properties.

### Host Cells

Any of a variety of recombinant host cells, each of which contains a nucleic acid sequence for use in expressing a desired protein, is also provided by the present invention. Exemplary prokaryotic and eukaryotic host cells include bacterial, mammalian, fungal, insect, yeast or plant cells.

Prokaryotic host cells include but are not limited to eubacteria such as Gram-negative or Gram-positive organisms (e.g., *E. coli* (HB101, DH5a, DH10 and MC1061); *Bacilli* such as *B. subtilis; Pseudomonas* species, such as *P. aeruginosa; Streptomyces* spp.; *Salmonella typhimurium;* or *Serratia marcescans.* As a specific embodiment, a KGF-2 protein(s) may be expressed in *E. coli.*

In addition to prokaryotic host cells, KGF-2 protein(s) may be expressed in glycosylated form by any one of a number of suitable host cells derived from multicellular organisms. Such host cells are capable of complex processing and glycosylation activities. In principle, any higher eukaryotic cell culture might be used, whether such culture involves vertebrate or invertebrate cells, including plant and insect cells.

Eukaryotic microbes such as filamentous fungi or yeast may be suitable hosts for the expression of a KGF-2 protein(s). *Saccharomyces cerevisiae,* or common baker's yeast, is the most commonly used among lower eukaryotic host microorganisms, but a number of other genera, species and strains are well known and commonly available.

Vertebrate cells may be used, as the propagation of vertebrate cells in culture (tissue culture) is a well-known procedure. Examples of useful mammalian host cell lines include but are not limited to monkey kidney CV1 line transformed by SV40 (COS-7), human embryonic kidney line (293 cells or 293 cells subcloned for growth in suspension culture), baby hamster kidney cells and Chinese hamster ovary cells. Other suitable mammalian cell lines include but are not limited to HeLa, mouse L-929 cells, 3T3 lines derived from Swiss, Balb-c or NIH mice, and BHK or HaK hamster cell lines. As a specific embodiment, a KGF-2 protein(s) may be expressed in COS cells or in baculovirus cells.

A host cell may be transfected and preferably transformed with a desired nucleic acid under appropriate conditions permitting expression of the nucleic acid. The selection of suitable host cells and methods for transformation, culture, amplification, screening and product production and purification are well known in the art (Gething and Sambrook (1981), Nature, 293:620-625 or, alternatively, Kaufman et al. (1985), Mol. Cell. Biol., 5(7):1750-1759, or U.S. Pat. No. 4,419,446, the disclosures of which are hereby incorporated by reference). For example, for mammalian cells without cell walls, the calcium phosphate precipitation method may be used. Electroporation, microinjection and other known techniques may also be used.

It is also possible that a desired protein may be produced by homologous recombination or with recombinant production methods utilizing control elements introduced into cells already containing DNA encoding a KGF-2 protein(s). Homologous recombination is a technique originally developed for targeting genes to induce or correct mutations in transcriptionally active genes (Kucherlapati (1989), Prog. in Nucl. Acid Res. and Mol. Biol., 36:301). The basic technique was developed as a method for introducing specific mutations into specific regions of the mammalian genome (Thomas et al. (1986), Cell, 44:419-428; Thomas and Capecchi (1987), Cell, 51:503-512 and Doetschman et al. (1988), Proc. Natl. Acad. Sci., 85:8583-8587) or to correct specific mutations within defective genes (Doetschrnan et al. (1987), Nature, 330:576-578). Exemplary techniques are described in U.S. Patent No. 5,272,071; WO 92/01069; WO 93/03183; WO 94/12650 and WO 94/31560.

### Culturing the Host Cells

The method for culturing each of the one or more recombinant host cells for production of a desired protein will vary depending upon many factors and considerations; the optimum production procedure for a given situation will be apparent to those skilled in the art through minimal experimentation. Such recombinant host cells are cultured in suitable medium and the expressed protein is then optionally recovered, isolated and purified from the culture medium (or from the cell, if expressed intracellularly) by appropriate means known to those skilled in the art.

Specifically, each of the recombinant cells used to produce a desired protein may be cultured in media suitable for inducing promoters, selecting suitable recombinant host cells or amplifying the gene encoding the desired protein. The media may be supplemented as necessary with hormones and/or other growth factors (such as insulin, transferrin or epidermal growth factor), salts (such as sodium chloride, calcium, magnesium and phosphate), buffers (such as HEPES), nucleosides (such as adenosine and thymidine), antibiotics (such as gentamicin), trace elements (defined as inorganic compounds usually present at final concentrations in the micromolar range), and glucose or another energy source. Other supplements may also be included, at appropriate concentrations, as will be appreciated by those skilled in the art. Suitable culture conditions, such as temperature, pH and the like, are also well known to those skilled in the art for use with the selected host cells.

The resulting expression product may then be purified to near homogeneity using procedures known in the art. Exemplary purification techniques are taught in published PCT Application Nos. WO 90/08771 and WO 96/11952, the disclosures of which are hereby incorporated by reference.

### Uses

KGF-2 protein(s) and chemically-modified derivatives thereof having biological activity (collectively, "KGF-2 protein product(s)") may be used as research reagents and as therapeutic and diagnostic agents. Thus, a KGF-2 protein product(s) may be used in *in vitro* and/or *in vivo* diagnostic assays to quantify the amount of KGF-2 in a tissue or organ sample.

For example, a KGF-2 protein product(s) can be used for identification of the receptor(s) for KGF-2 protein(s) in various body fluids and tissue samples using techniques known in the art (WO 90/08771).

This invention also contemplates the use of a KGF-2 protein product(s) in the generation of antibodies made against the KGF-2 protein product(s), including native KGF-2. One of ordinary skill in the art can use well-known, published procedures to obtain monoclonal and polyclonal antibodies or recombinant antibodies. Such antibodies may then be used to purify and characterize the KGF-2 protein product(s), including native KGF-2.

### Pharmaceutical Compositions

The present invention encompasses pharmaceutical preparations each containing therapeutically- or prophylatically-effective amounts of a KGF-2 protein product (s).

Pharmaceutical compositions each will generally include a therapeutically-effective or prophylatically-effective amount of a KGF-2 protein product(s) in admixture with a vehicle. The vehicle preferably includes one or more pharmaceutically and physiologically acceptable formulation materials in admixture with the KGF-2 protein product(s).

The primary solvent in a vehicle may be either aqueous or non-aqueous in nature. In addition, the vehicle may contain other pharmaceutically acceptable excipients for modifying or maintaining the pH (e.g., buffers such as citrates, phosphates, and amino acids such as glycine); osmolarity (e.g., mannitol and sodium chloride); viscosity; clarity; color; sterility; stability (e.g., sucrose and sorbitol); odor of the formulation; rate of dissolution (e.g., solubilizers or solubilizing agents such as alcohols, polyethylene glycols and sodium chloride); rate of release; as well as bulking agents for lyophilized formulation (e.g., mannitol and glycine); surfactants (e.g., polysorbate 20, polysorbate 80, triton, and pluronics); antioxidants (e.g., sodium sulfite and sodium hydrogen-sulfite); preservatives (e.g., benzoic acid and salicylic acid); flavoring and diluting agents; emulsifying agents; suspending agents; solvents; fillers; delivery vehicles; diluents and/or pharmaceutical adjuvants. Other effective administration forms such as parenteral slow-release formulations, inhalant mists, orally-active formulations, or suppositories are also envisioned.

The composition may also involve particulate preparations of polymeric compounds such as bulk erosion polymers (e.g., poly(lactic-co-glycolic acid) (PLGA) copolymers, PLGA polymer blends, block copolymers of PEG, and lactic and glycolic acid, poly(cyanoacrylates)); surface erosion polymers (e.g., poly(anhydrides) and poly(ortho esters)); hydrogel esters (e.g., pluronic polyols, poly(vinyl alcohol), poly(vinylpyrrolidone), maleic anhydride-alkyl vinyl ether copolymers, cellulose, hyaluronic acid derivatives, alginate, collagen, gelatin, albumin, and starches and dextrans) and composition systems thereof; or preparations of liposomes or microspheres. Such compositions may influence the physical state, stability, rate of *in vivo* release, and rate of *in vivo* clearance of the present proteins and derivatives. The optimal pharmaceutical formulation for a desired protein will be determined by one skilled in the art depending upon the route of administration and desired dosage. Exemplary pharmaceutical compositions are disclosed in Remington's Pharmaceutical Sciences, 18th Ed. (1990), Mack Publishing Co., Easton, PA 18042, pages 1435-1712; Gombotz and Pettit (1995), Bioconjugate Chem., 6:332-351; Leone-Bay, et al. (1995), Journal of Medicinal Chemistry, 38:4263-9269; Haas, et al. (1995), Clinical Immunology and Immunopathology, 76 (1) :93; WO 94/06457; WO 94/21275; FR 2706772 and WO 94/21235.

Specific sustained release compositions are available from the a variety of suppliers including Depotech Corp. (Depofoam^{™}, a multivesicular liposome); Alkermes, Inc. (ProLease^{™}, a PLGA microsphere). As used herein, hyaluronan is intended to include hyaluronan, hyaluronic acid, salts thereof (such as sodium hyaluronate), esters, ethers, enzymatic derivatives and cross-linked gels of hyaluronic acid, and chemically modified derivatives of hyaluronic acid (such as hylan). Exemplary forms of hyaluronan are disclosed in U.S. Patent Nos . 4, 582, 865, 4,605,691, 4, 636, 529, 4,713,448, 4, 716, 154, 4,716,224, 4,772,419, 4, 851, 521, 4,957,774, 4, 863, 907, 5,128,326, 5,202,431, 5, 336, 767, 5, 356, 883; European Patent Application Nos. 0 507 604 A2 and 0 718 312 A2; and WO 96/05845, the disclosures of which are hereby incorporated by reference. Suppliers of hyaluronan include BioMatrix , Inc., Ridgefield, NJ; Fidia S.p.A., Abano Terme, Italy; Kaken Pharmaceutical Co., Ltd., Tokyo, Japan; Pharmacia AB, Stockholm, Sweden; Genzyme Corporation, Cambridge, MA; Pronova Biopolymer, Inc. Portsmouth, NH; Calbiochem-Novabiochem AB, Lautelfingen, Switzerland; Intergen Company, Purchase, NY and Kyowa Hakko Kogyo Co., Ltd., Tokyo, Japan.

For treatment and/or prevention of oral indications, a liquid solution or suspension can be used in a manner similar to a mouthwash, where the liquid is swished around in the mouth so as to maximize treatment of lesions (United States Patent 5,102,870, the teachings of which are incorporated by reference). Longer contact with the mucosal surface can be attained by selecting a suitable vehicle which is capable of coating mucosa. Typical examples are pectin containing formulations such as Orabase Registered^{™} (Colgate-Hoyt Laboratories, Norwood, MA), sucralfate suspensions, Kaopectate and Milk of Magnesia. The formulation can also be a spreadable cream, gel, lotion or ointment having a pharmaceutically acceptable non-toxic vehicle or carrier. KGF-2 protein product(s) can also be incorporated into a slow dissolving lozenge or troche, a chewing gum base, or a buccal or slow delivery prosthesis hooked onto a back molar, for example. Therapeutic agents such as analgesics and anesthetics can be administered to alleviate pain and such as anti-infectictives, anti-bacterials, anti-fungals and antiseptics can be administered to prevent and/or treat secondary infection of the lesions.

Once the pharmaceutical composition has been formulated, it may be stored in sterile vials as a solution, suspension, gel, emulsion, solid, or a dehydrated or lyophilized powder. Such formulations may be stored either in a ready-to-use form or in a form (e.g., lyophilized) requiring reconstitution prior to administration.

In a specific embodiment, the present invention is directed to kits for producing a single-dose administration unit. The kits may each contain both a first container having a dried protein and a second container having an aqueous formulation. Kits included within the scope of this invention are single and multi-chambered pre-filled syringes; exemplary pre-filled syringes (e.g., liquid syringes, and lyosyringes such as Lyo-Ject®, a dual-chamber pre-filled lyosyringe) are available from Vetter GmbH, Ravensburg, Germany.

A KGF-2 protein product(s) may be applied in therapeutically- and prophylactically-effective amounts to organs or tissues specifically characterized by having damage to or clinically insufficient numbers of epithelium cells. It should be noted that KGF-2 protein product(s) formulations described herein may be used for veterinary as well as human applications and that the term "patient" should not be construed in a limiting manner.

The frequency of dosing the KGF-2 protein product(s) to a patient will depend on the disease and the condition of the patient, as well as the pharmacokinetic parameters of KGF-2 protein product(s) as formulated, and the route of administration. The KGF-2 protein product(s) may be administered once, administered daily, or administered with an initial bolus dose followed by a continuous dose or sustained delivery. It is also contemplated that other modes of continuous or near-continuous dosing may be practiced. For example, chemical derivatization may result in sustained release forms of the protein which have the effect of a continuous presence in the bloodstream, in predictable amounts, based on a determined dosage regimen.

A patient in need of stimulation (including cytoprotection, proliferation and/or differentiation) of epithelial cells may be administered an effective amount of a KGF-2 protein product(s) to elicit the desired response in the patient. The dosage regimen involved in a method of preventing or treating a specific condition generally will be determined by the attending physician, considering various factors which modify the action of drugs, e.g., the age, condition, body weight, sex and diet of the patient, the severity of any infection, the time of administration and other clinical factors. Appropriate dosages may be ascertained through use of established assays for determining dosages utilized in conjunction with appropriate dose-response data. Typical dosages will range from 0.001 mg/kg body weight to 500 mg/kg body weight, preferably up to 200 mg/kg body weight, more preferably 100 mg/kg body weight.

The KGF-2 protein product(s) may be administered via topical, enteral or parenteral administration including, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, and intrasternal injection and infusion. The KGF-2 protein product(s) may be administered via oral administration or administered through mucus membranes, that is, intranasally, sublingually, buccally or rectally for systemic delivery. The KGF-2 protein product(s) may be used once or administered repeatedly, depending on the disease and the condition of the patient. In some cases, the KGF-2 protein product(s) may be administered as an adjunct to other therapy and also with other pharmaceutical preparations.

Cell therapy (e.g., implantation of cells producing KGF-2 protein(s) can also be contemplated. This may include implanting into patients cells which are capable of synthesizing and secreting a biologically-active form of KGF-2 protein(s). Such cells producing KGF-2 protein(s) may be cells which do not normally produce KGF-2 protein(s) but which have been modified to produce KGF-2 protein(s), or which may be cells whose ability to produce KGF-2 protein(s) have been augmented by transformation with a polynucleotide suitable for the expression and secretion of KGF-2 protein(s). In order to minimize a potential immunological reaction in patients being administered KGF-2 protein(s) of a foreign species, it is preferred that the cells be of the same species as the patient (e.g., human), or that the cells may be encapsulated with material that provides a barrier against immune recognition, or that cells be placed into an immunologically-privileged anatomical location, such as in the testis, eye and central nervous system.

Human or non-human animal cells may be implanted in patients in biocompatible, semi-permeable polymeric enclosures or membranes to allow release of a KGF-2 protein(s) but prevent destruction of the cells by the patient's immune system or by other detrimental factors from the surrounding tissue. Alternatively, the patient's own cells, transformed *ex vivo* to produce KGF-2 protein(s), could be implanted directly into the patient without such encapsulation. The methodology for the membrane encapsulation of living cells is familiar to those of ordinary skill in the art, and the preparation of the encapsulated cells and their implantation in patients may be accomplished with known techniques (U.S. Patent Nos. 4,892,538; 5,011,472; and 5,106,627).

*In vivo* gene therapy can also be envisioned, wherein a nucleic acid sequence encoding a KGF-2 protein(s) is introduced directly into a patient. Efficient and long lasting gene transfer to hepatocytes is required for effective gene therapy for local expression of the protein to prevent and/or treat liver diseases and/or for secretion of the protein to prevent and/or treat diseases in other organs or tissues.

The DNA construct may be directly injected into the tissue of the organ to be treated, where it can be taken up *in vivo* and expressed, provided that the DNA is operable linked to a promoter that is active in such tissue. The DNA construct may also additionally include vector sequence from such vectors as an adenovirus vector, a retroviral vector, papilloma virus and/or a herpes virus vector, to aid uptake in the cells. Physical transfer may be achieved *in vivo* by local injection of the desired nucleic acid construct or other appropriate delivery vector containing the desired nucleic acid sequence, such as liposome-mediated transfer, direct injection (naked DNA), receptor-mediated transfer (ligand-DNA complex), or microparticle bombardment (gene gun). For the *in vivo* regeneration of hepatocytes in the liver, the use of Moloney retroviral vectors may be especially effective (Bosch, et al. (1996), Cold Spring Harbor, Gene Therapy Meeting, September 25-29, 1996; and Bosch, et al. (1996), Journal of Clinical Investigation, 98(12):2683-2687).

The following examples are included to more fully illustrate the present invention. It is understood that modifications can be made in the procedures set forth without departing from the the invention.

### EXAMPLES

Standard methods for many of the procedures described in the following examples, or suitable alternative procedures, are provided in widely recognized manuals of molecular biology such as, for example, Sambrook et al. (1989), *supra* and Ausubel et al.. (1990), *supra.* All chemicals are either analytical grade or USP grade.

### Example 1: Protein Production

The following example teaches the production of His rFGF10 and HFGF10.

### A. Preparation of DNA

### pAMG21 His rFGF10:

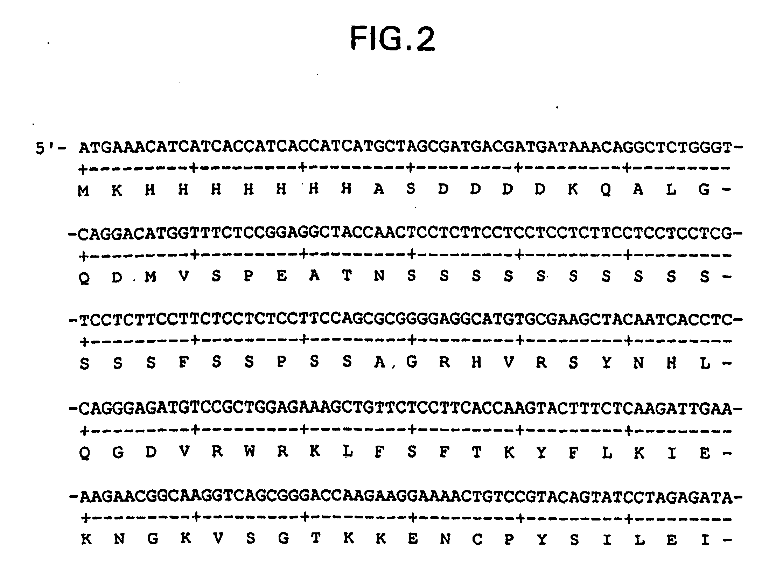

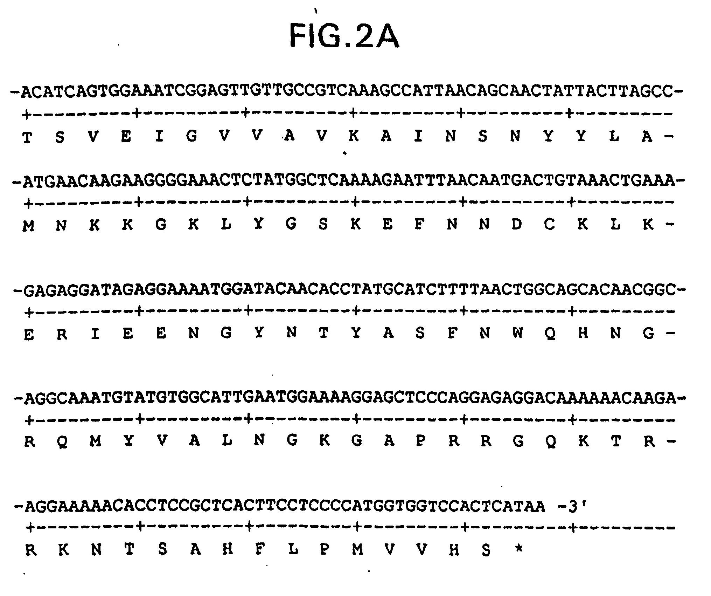

The plasmid pAMG21 His rFGF10 contains DNA encoding the amino acid sequence set forth in Figure 2. pAMG21 His rFGF10 was constructed as follows.

First, plasmid pAMG21 dN6 rFGF10 was constructed. For this construction, PCR was performed using mature rat FGF10 cDNA (Yamasaki et al. (1996), J. Biol. Chem., 271(27) :15918-15921, rFGF) in the vector pGEM-T (Promega, Madison, WI), termed pGEM-T rFGF10, as a template with the following 5' oligonucleotide primer (OLIGO#1), which incorporates an NdeI site, and 3' oligonucleotide primer (OLIGO#2) which incorporates a BamHI site:
OLIGO#1: (SEQ ID NO:7)
   5'-AAA CAA CAT ATG GTT TCT CCG GAG GCT ACC AAC TCC-3'
OLIGO#2: (SEQ ID NO:8)
   5'-AAA CAA GGA TCC TTT ATG AGT GGA CCA CCA TGG GG-3'
The PCR product generated in this reaction was purified and digested with restriction endonucleases NdeI and BamHI. The 525 base pair (bp) restriction-digested PCR product was purified from an agarose gel and ligated with a similarly purified 6 Kilobase (Kb) BamHI to NdeI pAMG21 vector DNA fragment. [The expression vector pAMG21 (ATCC accession no. 98113) contains appropriate restriction sites for insertion of genes downstream from a luxPR promoter (see U.S. Patent No. 5,169,318 for description of the lux expression system).] The resultant encoded rFGF10 protein differs from rFGF10 by deletion of the first 6 amino-terminal amino acid residues to a naturally occurring methionine residue, with the protein having the following amino-terminal (N-terminal) amino acid sequence: MVSPEAT.... (beginning at residue #43, Figure 2, Yamasaki et al. (1996), *supra*).

Next, the plasmid pAMG21 His rFGF10 was constructed using a pAMG21 His vector. The pAMG21 His vector differs from pAMG21 as follows: between the initiating methionine codon of pAMG21 (ATG) and the sequence that follows it (GTTAACG...), the following sequence is inserted "AAA CAT CAT CAC CAT CAC CAT CAT GCT AGC" which codes for "KHHHHHHHAS". The addition of the codons for Ala and Ser after the 7x His tag afford a convenient restriction site, NheI, for cloning.

The 4.7 Kb BstXI-NheI fragment of pAMG21 His plasmid vector was then ligated with the 1.8Kb BspEI-BstXI fragment of pAMG21 dN6 rFGF10 and the following oligonucleotide linkers OLIGO#3 and OLIGO#4 (NheI to BspEI).
OLIGO#3: (SEQ ID NO:9)
   5'-CTA GCG ATG ACG ATG ATA AAC AGG CTC TGG GTC AGG ACA TGG TTT CT-3'
OLIGO#4: (SEQ ID NO:10)
   5'-CCG GAG AAA CCA TGT CCT GAC CCA GAG CCT GTT TAT CAT CGT CAT CG-3'
The resultant encoded protein differs from dN6 rFGF10 by having a histidine tag (7x His) followed by an enterokinase cleavage site with the full-length (mature) N-terminal sequence (beginning at residue #37, Figure 1, Yamasaki et al. (1996), *supra*)*.* Twenty-two amino acids were added to the amino-terminus of the dN6 rFGF10 as follows: MKHHHHHHHASDDDDKQALGQD [MVSPKAT....].

*E*. *coli* host strain GM120 (ATCC accession no. 55764) has the lacIQ promoter and lacI gene integrated into a second site in the host chromosome of a prototrophic *E*. *coli* K12 host. Transformation of GM120 *E. coli* host with this ligation mixture and plating on Luria agar plates containing 40µg/ml kanamycin yielded recombinant bacterial colonies. A bacterial clone containing the correct recombinant plasmid was identified by PCR screening. Plasmid DNA was purified and sequenced to confirm the insert sequence. Growth of recombinant bacterial cultures to express the gene product is described below.

### pAMG21 hFGF10:

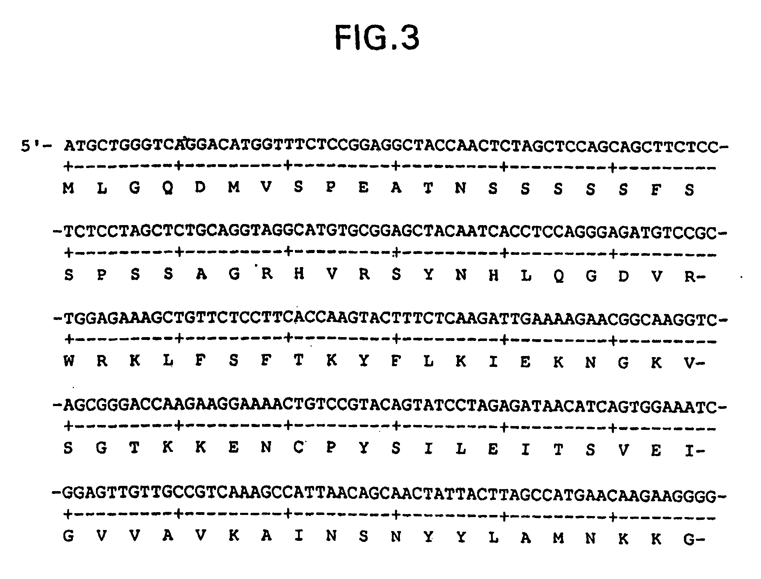

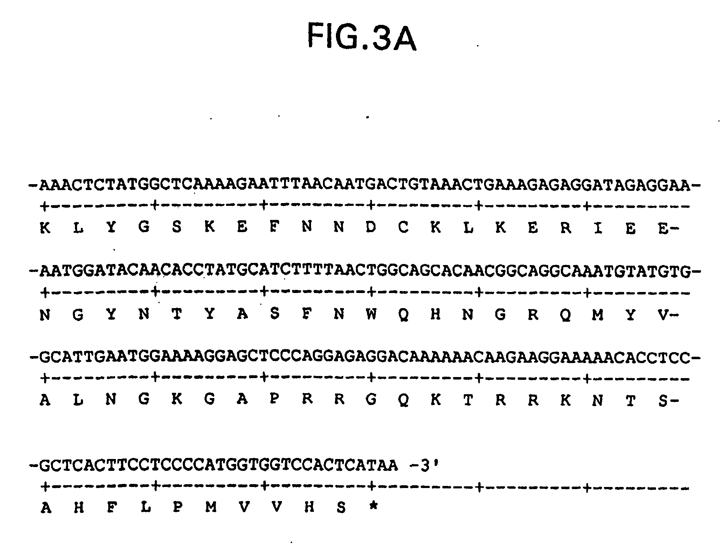

The plasmid pAMG21 hFGF10 contains DNA encoding the amino acid sequence set forth in Figure 3 (hFGF10). Thus hFGF10 has the sequence of Leu⁴⁰ to Ser²⁰⁸ of SEQ ID NO:2, as set forth above. pAMG21 hFGF10 was constructed as follows.

The plasmid pAMG21 dN20 hFGF10 contains a deletion of the DNA encoding the first 28 amino acids of the mature rFGF10 sequence resulting in the following N-terminal amino acid sequence: MSSPSSA..... (beginning at residue #65, Figure 2, Yamasaki et al. (1996), *supra*). pAMG21 dN20 hFGF10 was constructed as follows.

The 6 Kb BamHI-NdeI pAMG21 vector fragment was ligated to an NdeI-BamHI dN20 hFGF10 PCR product generated as follows: PCR was carried out using pGEM-T rFGF10 as the template and the following 5' oligonucleotide primer (OLIGO#5) which incorporates an NdeI site at the 5' end of the rFGF10 gene and deletes codons for the first 28 amino acids, and 3' oligonucleotide primer (OLIGO#6) which incorporates a BamHI site at the 3' end of the rFGF10 gene:
OLIGO#5: (SEQ ID NO:11)
   5'- AAA CAA CAT ATG TCT TCT.CCT TCC TCT GCA GGT AGG CAT GTG CGG AGC TAC AA -3'
OLIGO#6: (SEQ ID NO:12)
   5'- AAA CAA GGA TCC TTT ATG AGT GGA CCA CCA TGG GG -3'
This PCR product was purified, digested with restriction endonucleases NdeI and BamHI and, as described above, ligated to the 6 Kb BamHI-NdeI pAMG21 vector fragment.

Plasmid pAMG21 rFGF10 was created by ligation of the 6.5 Kb BspEI-NdeI fragment of pAMG21 His rFGF10 with the following oligonucleotide linkers OLIGO#11 and OLIGO#8 (NdeI to BspEI).
OLIGO#7: (SEQ ID NO:13)
   5'- TAT GCT GGG TCA GGA CAT GGT TTC T -3'
OLIGO#8: (SEQ ID NO:14)
   5'- CCG GAG AAA CCA TGT CCT GAC CCA GCA -3'
The resultant encoded protein differs from His rFGF10 by deletion of the 7x Histidine tag and restoration of the original mature amino terminal protein sequence (MLGQDM....).

A 4.8Kb BstXI-BspEI fragment of pAMG21 rFGF10 was ligated with the 1.8Kb fragment of pAMG21 dN20 hFGF10 PstI (introduced)-BstXI and the following OLIGO#13 and OLIGO#14 oligonucleotide linkers (PstI to BspEI) to delete eight serine codons from the rat sequence.
OLIGO#9: (SEQ ID NO:15)
   5'- CCG GAG GCT ACC AAC TCT AGC TCC AGC AGC TTC TCC TCT CCT AGC TCT GCA -3'
OLIGO#10: (SEQ ID NO:16)
   5'- GAG CTA GGA GAG GAG AAG CTG CTG GAG CTA GAG TTG GTA GCC T -3'

GM120 *E. coli* host cells were transformed with this pAMG21 hFGF10 ligation product, and plating on Luria agar plates containing 40µg/ml kanamycin yielded recombinant bacterial colonies. A bacterial clone containing the correct recombinant plasmid was identified by PCR screening. Plasmid DNA was purified and sequenced to confirm the insert sequence. Growth of recombinant bacterial cultures to express the gene product is described below.

### B. Production in E. coli

Cultures of recombinant GM120 *E. coli* cells containing the DNA sequence-confirmed plasmid of interest (pAMG21 His rFGF10 and pAMG21 hFGF10, respectively) are each grown to optimize expression of the introduced gene, as follows:

Five hundred milliliter flasks of Luria Broth plus Kanamycin were seeded with cells and grown at 30°C degrees from 10 to 16 hours. All 500 mL was added to a 9 L to 11 L of NZ amine-based media in a 15 L fermentor. All batches were grown at a pH of 7 and a dissolved oxygen level of >50%. Batches of cells containing pAMG21 His rFGF10 and containing pAMG21 hFGF10 were each grown and induced at 30°C. The batches were grown at a pH of 7 and a dissolved oxygen level of >50%. When optical cell density reached 10 ± 2, autoinducer was added to the fermentor and cells were allowed to grow for 12 hours. After 12 hours, the broth was chilled to less than 15°C, the fermentor was drained and the cells were collected by centrifugation. The cell paste was frozen.

### C. Purification

### His rFGF10:

His rFGF10 was purified from *E. coli* cell paste using three chromatography steps: S-Sepharose at pH 7.5, S-Sepharose at pH 8.5, and chelating (Ni)-Sepharose. Eighty grams of *E. coli* cell paste was homogenized in 10 volumes H₂O, then the cells were disrupted in a microfluidizer. The broken cell suspension was centrifuged for 3 hours at 15,300 x g. The supernatant containing soluble His rFGF10 was adjusted to 40 mM Tris-HCl, pH 7.5 by addition of 1 M Tris-HCl, pH 7.5, then applied to a 300 mL S-Sepharose FF column equilibrated in 40 mM Tris-HCl, pH 7.5. After washing the column extensively with equilibration buffer to remove unbound protein, the column was eluted with a 40-volume gradient from 0 to 2 M NaCl in 40 mM Tris-HCl, pH 7.5. Fractions eluting between 0.8 M and 1.0 M NaCl contained His rFGF10, which was detected as a 24 kDa band on SDS-PAGE. The identity of this band was confirmed by N-terminal sequencing. These fractions were pooled, diluted with H₂O to reduce the NaCl concentration to 0.4 M, and adjusted to pH 8.5 by addition of 1 M Tris-HCl, pH 9.2. This sample was applied to a 75 mL S-Sepharose HP column equilibrated in 40 mM Tris-HCl, pH 8.5. The column was washed with 40 mM Tris-HCl, pH 8.5 to remove unbound protein, then eluted with a 40-volume gradient from 0 to 2 M NaCl in the same buffer. Fractions eluting between 0.9 M and 1.1 M NaCl were pooled. The pooled fractions were applied to a 200 mL chelating (Ni)-Sepharose column equilibrated in 1 M NaCl, 40 mM Tris-HCl, pH 8.5, 1 mM imidazole. The column was washed with equilibration buffer to remove unbound protein, then eluted with a 20-volume gradient from 0 to 100 mM imidazole in 1 M NaCl, 40 mM Tris-HCl, pH 8.5, followed by a 20-volume gradient from 100 to 500 mM imidazole in the same buffer. His rFGF10 eluted between 100-200 mM imidazole. Fractions containing His rFGF10 were pooled, concentrated and buffer-exchanged to phosphate-buffered saline (PBS). Sample purity, analyzed by silver-stained or Coomassie Blue-stained SDS-gels, was estimated to be greater than 97%. Overall yield was 459 mg purified His rFGF10 from 80 g cell paste.

### hFGF10:

hFGF10 was purified using three chromatography steps: S-Sepharose at pH 7.5, Heparin-Sepharose at pH 7.5, and hydroxyapatite. One hundred grams *E. coli* cell paste containing hFGF10 was homogenized and disrupted exactly as described above. Following centrifugation at 15,300 x g for 3 hours, the supernatant containing soluble hFGF10 was adjusted to 40 mM Tris-HCl, pH 7.5, by addition of 1 M Tris-HCl, pH 7.5, then applied to a 300 mL S-Sepharose FF column equilibrated in 40 mM Tris-HCl, pH 7.5. After washing the column with equilibration buffer to remove unbound protein, the column was eluted with a 40-volume gradient from 0 to 2 M NaCl in 40 mM Tris-HCl, pH 7.5. Fractions eluting between 0.9 M and 1.1 M NaCl contained hFGF10, which was detected on SDS-PAGE. The identity of this band was confirmed by N-terminal sequencing. These fractions were pooled, diluted with 40 mM Tris-HCl, pH 7.5, to reduce the NaCl concentration to 0.4 M, and applied to a 60 mL Heparin-Sepharose column equilibrated in 40 mM Tris-HCl, pH 7.5. The column was washed with equilibration buffer to remove unbound protein, then eluted with an 80-volume gradient from 0 to 3 M NaCl in the same buffer. hFGF10 eluted between 1.0 M and 1.35 M NaCl. These fractions were pooled and dialyzed against 40 mM Tris-HCl, pH 7.5. The dialyzed sample was applied to a 50 mL hydroxyapatite column equilibrated in 40 mM Tris-HCl, pH 7.5. Following sample application the column was washed with equilibration buffer to remove unbound protein, then eluted with a 40-volume gradient from 0 to 0.5 M NaCl. Fractions eluting between 0.24 M and 0.44 M NaCl contained hFGF10. These fractions were pooled, concentrated, and buffer-exchanged to PBS. Sample purity was estimated to be greater than 97% by Coomassie Blue-stained SDS-gels. The yield of purified hFGF10 was 90 mg from 100 g cell paste.

### Example 2: In Situ Hybridization Survey

An *in situ* hybridization survey was undertaken to determine the sites of expression of KGF-2 in normal adult and embyronic rat tissues.

A segment of rat KGF-2 sequence, including the first 128 nucleotides of the published coding region (Genebank accession number: D79215) plus 100 nucleotides upstream from the translation initiation site, was amplified from a rat lung cDNA and cloned into pGEM-T (Promega). The linearized template was used to synthesize high specific activity ³³P-labeled riboprobes.

A panel of normal embryonic and adult rat tissues were fixed in 4% paraformaldehyde, embedded in paraffin and sectioned at 5 µm. Prior to *in situ* hybridization, tissues were permeabilized with 0.2M HCl, followed by digestion with Proteinase K, and acetylation with triethanolamine and acetic anhydride. Sections were hybridized with ³³P-labeled riboprobes overnight at 55°C, then subjected to a high stringency wash in 0.1X SSC at 60°C. Slides were dipped in NTB2 film emulsion (Eastman Kodak, Rochester, NY), exposed at 4°C for 2-3 weeks, developed and counterstained. Sections were examined with darkfield and standard illumination to allow simultaneous evaluation of tissue morphology and hybridization signal.

Overall, the KGF-2 probe produced a clear distribution of signal in the tissue sections from both embryo and adult, with little or no background signal. In the late stage embryo, the majority of the KGF-2 signal was observed in cells with a mesenchymal appearance, with little epithelial expression noted (Table 2). In the adult, unlike the embryo, KGF-2 expression was detected in both epithelial and mesenchymal tissues (Table 2).

**TABLE 2: In Situ Hybridization**

| | |
|---|---|
| In Situ Hybridization of KGF-2 mRNA in Embryonic Day 17.5 Rats | Expression in submaxillary gland, stomach, intestine, cochlea, tooth bud and kidney. |
| In Situ Hybridization and RNase Protection Assay of KGF-2 in Adult Rat | Expression in salivary gland, dermal papillae, esophogus, stomach, small and large intestines and breast ducts. |

### Example 3: His rFGF10 Stimulated Proliferation and Differentiation of Epithelial Cells

Eighteen female BDF1 mice were divided into 6 groups of 3 mice each (1 treated and 1 control group at each of 3 time points). The first two groups received 5 mg/kg His rFGF10 or the buffer control IV for 1 day; the second two groups received 5 mg/kg His rFGF10 or the buffer control IV daily for 3 days; and the third two groups received 5 mg/kg His rFGF10 or the buffer control IV daily for 7 days. All mice were injected with 50 mg/kg BrdU one hour prior to harvest, radiographed and sacrificed. Body and selected organ weights (including all segments of small intestine) were taken, blood was drawn for hematology and serum chemistries, and organs were harvested for histologic analysis and BrdU labeling.

An additional study was done using athymic nude mice. In this study, 5 mg/kg/day His rFGF10 was injected subcutaneously in the flank of five female nude mice for 8 days. Another five female nude mice received the buffer control. All mice were injected with 50 mg/kg BrdU one hour prior to harvest and were sacrificed. Body and selected organ weights were taken, blood was drawn for hematology and serum chemistries, and organs were harvested for histologic analysis and BrdU labeling.

BrdU immunohistochemistry was performed on 4 µm thick sections of zinc formalin fixed, paraffin-embedded tissue. BrdU was detected with a rat monoclonal antibody (MAb) to BrdU (Accurate Chemical, Westbury, NY), followed by a biotinylated anti-rabbit/anti-mouse secondary cocktail (BioTek Solutions, Inc., Santa Barbara, CA) and an ABC tertiary coupled to alkaline phosphatase (BioTek). The staining reaction was visualized with red chromagen (BioTek).

H&E and BrdU stained sections were examined. Significant histologic findings are summarized in Table 3. In addition to findings listed below, nude mice injected subcutaneously with His rFGF10 had localized epidermal and sebaceous gland hyperplasia at the injection site, as well as normalization of follicular morphology with hair shafts evident above the epidermal surface.

**TABLE 3: Histology Summary by Organ Systems**

| ORGAN SYSTEM | CONTROL | His rFGF10 DAY 1 | His rFGF10 DAY 3 | His rFGF10 DAY 7 |
|---|---|---|---|---|
| Hematopoietic | NSF* | NSF | There was a mild-moderate increase in splenic red pulp megakaryocytes. | There was a moderate-marked increase in splenic red pulp megakaryocytes |
| Gastro-intestinal | NSF | There was a marked increase in BrdU positive cells in the mucosa of the glandular and non-glandular stomach, as well as in pancreatic ductal epithelial cells. | The gastric mucosa (primarily non-glandular) exhibited moderate hyperplasia. There was mild-moderate goblet cell hyperplasia throughout the GI tract, particularly in the colon. There was mild-moderate pancreatic ductal hyperplasia. | The gastric mucosa (primarily non-glandular) exhibited moderate hyperplasia (but less than at 3 days). There was moderate-marked goblet cell hyperplasia throughout the GI tract, particularly in the colon. Intestinal villi were moderately elongated and intestinal and colonic crypts were moderately hyperplastic. There was mild-moderate pancreatic ductal hyperplasia. |
| Hepatobiliary | NSF | There was a marked increase in BrdU positive hepatocytes and bile duct epithelial cells. | There was a moderate to marked increase in hepatocellular mitotic figures, a moderate increase in hepatocellular BrdU labeling, and moderate-marked hepatocellular microvesiculation. | There was a slight increase in hepatocellular mitotic figures, and moderate hepatocellular microvesiculation. |
| Urinary | NSF | There was a marked increase in BrdU positive transitional epithelial cells in the urinary bladder and renal pelvis. | There was moderate-marked transitional epithelial hyperplasia in the urinary bladder and renal pelvis. | There was moderate transitional epithelial hyperplasia in the urinary bladder and renal pelvis. |
| Endocrine | NSF | There was a mild-moderate increase in BrdU positive thyroid follicular epithelial cells. | There was mild thyroid follicular hyperplasia. | There was mild thyroid follicular hyperplasia. |
| Reproductive | NSF | There was a marked ' increase in BrdU positive mammary gland ductal epithelial cells. | The mammary gland exhibited moderate-marked cystic ductular hyperplasia and a moderate increase in ductal epithelial cell BrdU labeling. | The mammary gland exhibited moderate ductular hyperplasia with relatively marked cystic change. |

| | | | | |
|---|---|---|---|---|
| * No significant findings | | | | |

### Example 4: Trophic Response in the small Intestines of Normal Mice

Female BDF1 mice were injected intraperitonealy (IP) with either His rFGF10 (5 mg/kg) or saline for three consecutive days. Twenty-three hours after the last injection, each mouse was sacrificed, the intestines were removed and flushed with cold saline, hung under uniform tension and divided into duodenum, jejunum and ileum. The wet weights of these tissues were taken and expressed as percent of body weight, and an unpaired t-test was used to compare His rFGF10 treated to saline treated. There was a 26% increase in small intestine wet weight.

### Example 5: Trophic Effect in Rats with 80% Short Bowel Resection

For this study 80% of the small bowel (5 cm distal to the ligament of Treitz to 5 cm proximal to the ileocecal valve) was surgically resected and the animals allowed to recover for 4 weeks. Animals were than treated for 7 days with either saline or His rFGF10 at 5 mg/kg subcutaneously. The animals were sacrificed and the remaining intestine removed, flushed with cold saline and hung with uniform tension of 10g. Total length was determined and the gut was sectioned as follows: first 1/3 designated duodenum, second 1/3 jejunum and last 1/3 ileum. A portion of each was taken for histology and for biochemical analysis. The portion for histology was processed into paraffin, cut in cross section and stained with hematoxylin and eosin. Image analysis was used to measure mucosal thickness and crypt depth in the ileum and jejunum, and this data was used to calculate the villus height where mucosal thickness - crypt depth = villus height. There was significant (p<0.01) increase in mucosal thickness due to an increase in villus height.

### Example 6: Crypt Survival in the Small Intestines of Irradiated Mice

Female BDF1 mice were dosed with 5 mg/kg/day His rFGF10 or vehicle for 3 days and subsequently irradiated with 12 Gy from a cesium source. Four days later the mice were injected with 50 mg/kg of BrdU IP one hour prior to euthanasia. The intestines were removed, flushed with cold saline, hung under uniform tension using a 5 gm weight and divided into duodenum, jejunum and ileum. These segments were weighed, fixed in formalin and blocked into paraffin for cross sectioning. Sections were stained with antibodies to BrdU and labeled crypts counted using a light microscopy. Positive crypt were identified as having 3 or more labeled nuclei/crypt. Pretreatment increased crypt survival by 45%, while post-treatment was as significantly effective.

### Example 7 Not part of the invention: Effect of pre-treatment with His rFCF10 in a Neonatal Rat Model of Cytosine Arabinoside (ARA-C) Chemotherapy-Induced Alopecia

Five litters of neonatal rats (approximately 10-15 pups per group) were injected IP with His rFGF10 at 5 mg/kg/day or PBS at 5, 6, and/or 7 days of age (experimental days -3, -2, and/or -1). On experimental days 0-5 (8-12 days of age), all pups were injected IP with 30 mg/kg cytosine arabinoside (ARA-C).

Pups were scored 0-4 for hair coverage and hair density by three independent observers blinded to the treatment groups on experimental days 6, 8, 11, 13 and 15. Scores for density and coverage were calculated for each rat pup daily, scores were added, and mean sums for each treatment group were calculated and tested for statistical significance at each timepoint using the Kruskal-Wallis nonparametric rank sums test.

His rFGF10 at all dosing regimes only induced a significant degree of protection from ARA-C-induced alopecia on experimental day 8, although there was a His rFGF10-induced trend toward ARA-C protection at all other timepoints.

### Example 8: Chemotherapy-induced Mucositis Model

Mice (15/group) treated with 5-fluorouracil, were administered with vehicle alone (saline), and with His rFGF10.
Control group: On days -2 to 0, a group of mice were administered saline on days -2 to 0, and injected intraperitoneally with 5-fluorouracil (5-FU, 60 mg/kg/day) on days 1 to 4.
His rFGF10 pretreatment: On days -2 to 0, a group of mice were injected subcutaneously with His rFGF10 (5 mg/kg). On days 1 to 4; the mice were injected intraperitoneally with 5-fluorouracil (5-FU, 60 mg/kg/day).

The effects on body weights and survival of the various groups were analyzed. Body weights were taken daily from day 0 to termination on day 30. Mice were examined visually twice/day for signs of morbidity for 30 days and all moribund animals were euthanized. The percent change in body weight on day 6 nadir is set forth in the Table 4.

**TABLE 4: Effects of His rFGF10 on Body Weight**

| Protein | % Change in Body Weight |
|---|---|
| Saline | -11.6 |
| His rFGF10 pretreatment | -12.2 |

Pretreatment with His rFGF10 also did demonstrate an increase in survival.

### Example 9: Radiation Mortality-Model

Pretreatment with recombinant human KGF-2 (having the sequence of Cys³⁷ to Servos of SEQ ID NO. 2 and being prepared generally in accordance with the teachings of WO 96/25422, rhuKGF-2), either combined with G-CSF or saline post-BMT, significantly improved survival when compared to G-CSF alone or to saline controls. The numbers of small intestinal proliferating crypts were increased 4 days following 12 Gy in rhuKGF-2 pretreated mice and the BMT procedure did not alter the magnitude of this rhuKGF-2-induced response.
Additionally, rhuKGF-2 did not alter the hematopoietic recovery kinetics of mice BMTed following non-lethal (9 Gy) TBI. rhuKGF-2 pretreatments prevented mortality in lethally-irradiated (12 Gy) mice transplanted with peripheral blood progenitor cells (1 x 10⁷ PBPCs) when used alone or in combination with G-CSF (100 µg/kg/day, SC, days 1 to 10). Similarly to the BMT experiments, no control PBPC transplanted mice survived and mice treated with G-CSF alone had an improvement. rhuKGF-2 pretreatments also did not alter the kinetics of hematopoietic recovery following PBPC transplantation, in the presence or absence of G-CSF, after non-lethal (8.5 Gy) TBI. When rhuKGF-2 was given both prior to and after lethal irradiation and PBPCs, it neither impaired the improved survival seen in mice treated with rhuKGF-2 pretreatments alone, nor did it alter the kinetics of hematopoietic reconstitution following non-lethal irradiation.

### Example 10: Radiation-induced Mucositis Model

Mucositis is induced in mice with 12 Gy of whole-body radiation. Mice are treated daily with 5 mg/kg/day of recombinant human KGF-2 (prepared generally in accordance with the teachings of WO 96/25422, rhuKGF-2) beginning on the day before radiation and continuing to day three post-radiation. Four days after radiation, the mice are necropsied and the number of proliferating crypts (containing BUdR-positive cells) are counted.

rhuKGF-2 treatment increases the number of proliferating crypts in the duodenum, proximal and distal jejunum of the small intestine relative to non-rhuKGF-2 treated animals. rhuKGF-2 is also able to decrease body weight loss in the irradiated mice.

### Example 11: Adriamycin-induced Mucositis Model

Mucositis is induced in mice with a single intraperitoneal dose of Adriamycin at 24 mg/kg. Mice are treated daily with 1 mg/kg/day of rhuKGF-2 beginning on the day before radiation and continuing to day three post-radiation. Four days after radiation, the mice are necropsied and the number of proliferating crypts (containing BUdR-positive cells) are counted.

rhuKGF-2 treatment increases the number of proliferating crypts in the duodenum, jejunum and ileum relative to non-rhuKGF-2 treated animals.

### Example 12: Colitis Model

In two groups of 10 animals each, colitis is induced by colonic instillation of 2,4,6-trinitrobenzenesulfonic acid in ethanol at a dose of 50 mg/kg body weight. To determine if rhuKGF-2 acts through a protective mechanism, one group of rats (group A) is pretreated with rhuKGF-2 or vehicle at 24 hours and at 1 hour prior to induction of colitis at a dose of 5 mg/kg (i.p.) and the animals are sacrificed 8 hours after injury. To assess potential healing effects, rhuKGF-2 or vehicle (same dosage, i.p.) is injected in a second group (group B) 24 hours after induction of colitis and treatment is continued daily for 1 week. Tissue damage is examined microscopically and is expressed as percentage of ulcerations or erosions.

Animals which are treated with rhuKGF-2 after induction of colitis (group B) show significantly less ulcerations compared to the control group (group A). In animals treated prior to induction of colitis, there are erosions, but no ulcers are seen due to the short study period of 8 hours, and the erosions are not significantly different from those seen in the control group (group A).

### Example 13: Dextran Sulfate-induced Colitis Model

Study 1: Rats are fed 4% and 6% DSS in water for 1 week. At the end of the second week the distal 4 cm of the colon is preserved. Eight sections at 0.5 cm intervals are prepared and stained with H & E. The percent of each colon section with necrosis (destruction of the glandular structure) is assessed in a randomized and coded fashion.

Study 2: Rats are given IP vehicle or rhuKGF-2 (1 mg/kg/day) and fed either water or 4% dextran sulfate sodium for 14 days. The colonic sections are stained with PAS.

Dextran sulfate sodium appears to induce a dose-related increase in colonic mucosal necrosis. rhuKGF-2 administered at 1 mg/kg/day for 14 days increases colonic mucin production in the control group as well as in the dextran sulfate sodium-treated rats.

### Example 14: Rat Cirrhosis Model

Male Sprague-Dawley rats weighing between 150 and 175 gms are used. Animals are exposed to phenobarbitol (0.35 mg/ml) in their drinking water for the duration of the study. Animals are dosed weekly with CC14 in a corn oil vehicle while under light isoflurance anesthesia. The initial dose of CC14 is 40 µl per rat. The dose is adjusted weekly, up or down in 40 µl increments based on weight gain. Ten control animals are exposed to phenobarbitol in their drinking water and lavaged weekly with corn oil vehicle. Liver function is assessed by measurement of bromosulphopthylein (BSP) clearance, serum transaminase levels and serum albumin levels. At the time of sacrifice, livers are removed, weighed and processed for determination of hydroxyproline levels as an indicator of collagen deposition and fibrosis.

Animals are maintained on the above cirrhosis induction protocol for 11 weeks. In the eleventh week, animals are randomized into control and rhuKGF-2 treatment groups. rhuKGF-2 is given once per day by subcutaneous injection at a dose of 1 mg/kg, for a total of 15 days. After 15 days of rhuKGF-2 treatment, the animals are euthanized.

Rats in which cirrhosis is induced with CC14 show an elevation in serum BSP concentration, reflecting impaired liver clearance of this agent. Rats treated with rhuKGF-2 have a lower BSP serum level than untreated animals, suggesting an improved liver function. Rats made cirrhotic by CC14 show an elevation in SGPT which is reversed by rhuKGF-2 treatment. rhuKGF-2 treatment is able to elevate serum albumin. rhuKGF-2 treatment results in an increased liver-to-body weight ratio, reflecting compensatory liver growth.

### Example 15: Hepatectomy Model

Rats subjected to a 70% partial hepatectomy recover their original liver mass more quickly when treated with 1 mg/kg/d rhuKGF-2 than when compared to untreated animals.

### Example 16: Acute hepatotoxicity Models

In acute hepatotoxicity models, rhuKGF-2 treatment (1 mg/kg either prior to or 3 hr after the inciting agent) blunts increases in serum transaminase levels in rats with acute hepatic failure induced with either carbon tetrachloride, acetaminophen or galactosamine. Pretreatment with rhuKGF-2 also prevents a decrease in liver clearance functions after acetaminophen, as measured by sulfobromophthalein (BSP) clearance.

### Example 17: In Vivo Retro-viral-Mediated Gene Transfer Model

Mice are intraveneously administered with rhuKGF-2 (1-5 mg/kg). 48 hours after intravenous injection of rhuKGF-2, murine hepatocyte proliferation increases, compared to non-stimulated livers, and returns to normal proliferative levels. No modules or microscopic abnormalities are noted either acutely or after 5 months.

When rhuKGF-2 treatment is followed by intravenous injection of high titer *E. coli LacZ* expressing Moloney retroviral vectors (1 x 108 cfu.ml), β-galactosidase expression increases with a percentage of the hepatocytes being transduced. Several months later, a portion of the transduced hepatocytes remain X-gal positive.

### Example 18: In Vivo Model of Diabetes

Male rats weighing 200 to 260 grams at study initiation are used in this model (WO 9611950). Diabetes is induced by a single intravenous injection of streptozotocin at 50 mg of streptozotocin in sodium citrate buffer per kg of body weight. Non-diabetic control rats receive a single intravenous injection of sodium citrate buffer for control purposes. rhuKGF-2 is administered daily as a subcutaneous injection. The rhuKGF-2 dose is 3 or 5 mg/kg/day, depending upon the experiment.

In the first experiment, rhuKGF-2 therapy is initiated two days before diabetes is induced and is continued after the induction of diabetes for a total of eight injections. Those diabetic rats which are treated with rhuKGF-2 prior to diabetes induction, and for which rhuKGF-2 is also continued after the induction, show symptoms indicative of a milder form of diabetes. Thus, rhuKGF-2 therapy either partially prevents induction of the disease or restores insulin-producing islet cells after streptozotocin-induced beta cell destruction.

In the second and third experiments, rhuKGF-2 therapy administered subcutaneously is initiated one day after the induction of diabetes with streptozotocin. In the second study, fasting water intake and urine output are significantly less in the rhuKGF-2-treated diabetic rats when compared to diabetic rats on day 9, which is further indicative of amelioration of the disease condition. In the third study, rhuKGF-2 therapy is able to increase the total content of insulin and C-peptide in the pancreas of diabetic rats when compared to diabetic rats treated with sodium chloride solution.

In the fourth experiment, a 7-day course of rhuKGF-2 therapy is initiated 7 days after streptozotocin treatment and the animals are then followed for an additional 12 weeks. In all experiments, except for the fourth experiment, blood glucose levels, urine glucose levels and urine volume are used as end points for analysis. Additionally, water intake, urine C-peptide levels, or total pancreatic insulin and C-peptide content are measured in some experiments. In the fourth experiment, the only assessed endpoint is blood glucose.

Because a large fraction of insulin is removed from the circulation by the liver, measurement of peripheral insulin concentrations reflects post-hepatic metabolism events rather than insulin secretion from the pancreas. Therefore, measurements of C-peptide are often made and used as a peripheral marker of insulin secretion. C-peptide is produced from the processing of pro-insulin to insulin. Insulin and C-peptide are secreted from the beta cells in equimolar amounts, and only a small amount of C-peptide is extracted by the liver.

STZ-treated animals from both groups receiving rhuKGF-2 have significant declines in blood glucose during the rhuKGF-2 dosing period.

While the present invention has been described above both generally and in terms of preferred embodiments, it is understood that other variations and modifications will occur to those skilled in the art in light of the description above.

## Claims

1. An *in vitro* method of stimulating the production of epithelial cells selected from cells within the pancreas, liver and gastrointestinal tract, comprising contacting such cells with an effective amount of KGF-2 protein(s).

2. The method according to Claim 1, wherein said KGF-2 protein(s) has an amino acid sequence comprising amino acids Cys³⁷ to Ser²⁰⁸ of SEQ ID NO: 2 or a variant sequence thereof, wherein said variant comprises deletions, insertions, substitutions and/or additions and retains the biological activity of the mature KGF-2 protein.

3. The method according to Claim 2 wherein said variant sequence comprises an amino acid sequence in excess of eighty percent (80%), ninety percent (90%), ninety-five percent (95%) or ninety-nine percent (99%) homologous to amino acids Cys³⁷ to Ser²⁰⁸ of SEQ ID NO: 2 and wherein said variant comprises deletions, insertions, substitutions and/or additions and retains the biological activity of the mature KGF-2 protein.

4. The method according to Claim 2, wherein said KGF-2 protein(s) is fused with all or part of the constant domain of the heavy or light chain of human immunoglobulin.

5. The method according to any one of Claims 1 to 4, wherein said KGF-2 protein(s) is chemically modified by linking it to a polymer.

6. The method according to Claim 5, wherein said KGF-2 protein(s) is chemically modified by conjugation with a water soluble polymer.

7. The method according to any one of Claims 1 to 6, wherein prior to administration said KGF-2 protein(s) is reconstituted from a dehydrated or lyophilized powder.

8. Use of an effective amount of KGF-2 protein(s) for the production of a medicament for the prevention or treatment of a condition in a patent suffering from a condition selected from gastric ulcers, duodenal ulcers, erosions of the stomach and esophagus, erosive gastritis, esophagitis, or esophageal reflux, inflammatory bowel disease, radiation or chemotherapy-induced gastrointestinal toxicity, hepatic cirrhosis, fulminant liver failure, acute viral hepatitis, toxic insult to the liver, and diabetes.

9. The use according to Claim 8, wherein said KGF-2 protein(s) has an amino acid sequence comprising amino acids Cys³⁷ to Ser²⁰⁸ of SEQ ID NO:2 or a variant sequence thereof, wherein said variant comprises deletions, insertions, substitutions and/or additions and retains the biological activity of the mature KGF-2 protein.

10. The use according to Claim 11, wherein said variant sequence comprises an amino acid sequence in excess of eighty percent (80%), ninety percent (90%), ninety-five percent (95%) or ninety-nine percent (99%) homologous to amino acids Cys³⁷ to Ser²⁰⁸ of SEQ ID NO:2 and wherein said variant comprises deletions, insertions, substitutions and/or additions and retains the biological activity of the mature KGF-2 protein.

11. The use according to Claim 9, wherein said KGF-2 protein(s) is fused with all or part of the constant domain of the heavy or light chain of human immunoglobulin.

12. The use according to any one of Claims 8 to 11, wherein said KGF-2 protein(s) is chemically modified by linking it to a polymer.

13. The use according to Claim 12, wherein said KGF-2 protein(s) is chemically modified by conjugation with a water soluble polymer.

14. The use according to any one of Claims 8 to 13, wherein prior to administration said KGF-2 protein(s) is reconstituted from a dehydrated or lyophilized powder.

15. The use according to any one of Claims 8 to 14, wherein said KGF-2 protein(s) is administered via a topical, enteral or parenteral route.

## Patentansprüche

1. Ein *in vitro*-Verfahren zur Stimulierung der Produktion von Epithelzellen, die aus Zellen innerhalb des Pankreas, der Leber und des Magen-DarmTraktes ausgewählt sind, das das Inkontaktbringen solcher Zellen mit einer wirksamen Menge von KGF-2 Protein(en) umfasst.

2. Das Verfahren gemäß Anspruch 1, worin besagte(s) KGF-2 Protein(e) eine Aminosäuresequenz aufweist (aufweisen), die die Aminosäuren Cys³⁷ bis Ser²⁰⁸ der SEQ ID NO: 2 oder eine Variantensequenz davon umfasst, wobei besagte Variante Deletionen, Insertionen, Substitutionen und/oder Additionen umfasst und die biologische Aktivität des reifen KGF-2 Proteins beibehält.

3. Das Verfahren gemäß Anspruch 2, worin besagte Variantensequenz eine Aminosäuresequenz umfasst, die mehr als achtzig Prozent (80 %), neunzig Prozent (90 %), fünfundneunzig Prozent (95 %) oder neunundneunzig Prozent (99 %) homolog zu den Aminosäuren Cys³⁷ bis Ser²⁰⁸ von SEQ ID NO: 2 ist und wobei besagte Variante Deletionen, Insertionen, Substitutionen und/oder Additionen umfasst und die biologische Aktivität des reifen KGF-2 Proteins beibehält.

4. Das Verfahren gemäß Anspruch 2, worin besagte(s) KGF-2 Protein(e) mit der ganzen oder einem Teil der konstanten Domäne der schweren oder leichten Kette eines menschlichen Immunglobulins fusioniert ist (sind).

5. Das Verfahren gemäß einem der Ansprüche 1 bis 4, worin besagte(s) KGF-2 Protein(e) durch Bindung desselben an ein Polymer chemisch modifiziert ist (sind).

6. Verfahren gemäß Anspruch 5, worin besagte(s) KGF-2 Protein(e) durch Konjugation mit einem wasserlöslichen Polymer chemisch modifiziert ist (sind).

7. Das Verfahren gemäß einem der Ansprüche 1 bis 6, worin besagte(s) KGF-2 Protein(e) vor der Verabreichung aus einem dehydrierten oder lyophilisierten Pulver rekonstituiert wird (werden).

8. Verwendung einer wirksamen Menge von KGF-2 Protein(en) zur Herstellung eines Medikaments zur Prävention oder Behandlung eines Zustandes in einem Patienten, der an einem Zustand leidet, der aus Magengeschwüren, Zwölffingerdarmgeschwüren, Erosionen des Magens und des Esophagus, erosiver Gastritis, Esophagitis oder Esophagusreflux, entzündlicher Darmerkrankung, durch Strahlungs- oder Chemotherapie induzierter Magen-Darm-Toxizität, Leberzirrhose, fulminantem Leberversagen, akuter viraler Hepatitis, toxischer Leberverletzung und Diabetes ausgewählt ist.

9. Die Verwendung gemäß Anspruch 8, worin besagte(s) KGF-2 Protein(e) eine Aminosäuresequenz aufweist (aufweisen), die die Aminosäuren Cys³⁷ bis Ser²⁰⁸ von SEQ ID NO: 2 oder eine Variantensequenz davon umfasst, wobei besagte Variante Deletionen, Insertionen, Substitutionen und/oder Additionen umfasst und die biologische Aktivität des reifen KGF-2 Proteins beibehält.

10. Die Verwendung gemäß Anspruch 11, wobei besagte Variantensequenz eine Aminosäuresequenz umfasst, die mehr als achtzig Prozent (80 %), neunzig Prozent (90 %), fünfundneunzig Prozent (95 %) oder neunundneunzig Prozent (99 %) homolog zu den Aminosäuren Cys³⁷ bis Ser²⁰⁸ von SEQ ID NO: 2 ist und wobei besagte Variante Deletionen, Insertionen, Substitutionen und/oder Additionen umfasst und die biologische Aktivität des reifen KGF-2 Proteins beibehält.

11. Die Verwendung gemäß Anspruch 9, worin besagte(s) KGF-2 Protein(e) an die gesamte oder einen Teil der konstanten Domäne der schweren oder leichten Kette eines menschlichen Immunglobulins fusioniert ist (sind).

12. Die Verwendung gemäß einem der Ansprüche 8 bis 11, worin besagte(s) KGF-2 Protein(e) durch die Bindung desselben an ein Polymer chemisch modifiziert ist (sind).

13. Die Verwendung gemäß Anspruch 12, worin besagte(s) KGF-2 Protein(e) durch Konjugation mit einem wasserlöslichen Polymer chemisch modifiziert ist (sind).

14. Die Verwendung gemäß einem der Ansprüche 8 bis 13, worin besagte(s) KGF-2 Protein(e) vor der Verabreichung aus einem dehydrierten oder lyophilisierten Pulver rekonstituiert wird (werden).

15. Die Verwendung gemäß einem der Ansprüche 8 bis 14, worin besagte(s) KGF-2 Protein(e) topisch, enteral oder parenteral verabreicht wird (werden).

## Revendications

1. Méthode *in vitro* de stimulation de la production de cellules épithéliales choisies parmi des cellules à l'intérieur du pancréas, du foie, du tractus gastro-intestinal, comprenant la mise en contact de ces cellules avec une quantité efficace d'une/de protéine(s) KGF-2.

2. Méthode selon la revendication 1, dans laquelle ladite/lesdites protéine(s) KGF-2 a/ont une séquence d'acides aminés comprenant les acides aminés Cys³⁷ à Ser²⁰⁸ de SEQ ID N°2 ou une variante de séquence de celle-ci, dans laquelle ladite variante comprend des suppressions, des insertions, des substitutions et/ou des ajouts et retient l'activité biologique de la protéine KGF-2 mature.

3. Méthode selon la revendication 2, dans laquelle ladite variante de séquence comprend une séquence d'acides aminés étant à plus de quatre-vingts pour cent (80 %), quatre-vingt-dix pour cent (90 %), quatre-vingt-quinze pour cent (95 %) ou quatre-vingt-dix-neuf pour cent (99 %) homologue aux acides aminés Cys³⁷ à Ser²⁰⁸ de SEQ ID n°2 et dans laquelle ladite variante comprend des suppressions, des insertions, des substitutions et/ou des ajouts et retient l'activité biologique de la protéine KGF-2 mature.

4. Méthode selon la revendication 2, dans laquelle ladite/lesdites protéine(s) KGF-2 est/sont fusionnée(s) avec tout ou partie du domaine constant de la chaîne lourde ou légère de l'immunoglobuline humaine.

5. Méthode selon l'une quelconque des revendications 1 à 4, dans laquelle ladite/lesdites protéine(s) KGF-2 est/sont modifiée(s) chimiquement par liaison avec un polymère.

6. Méthode selon la revendication 5, dans laquelle ladite/lesdites protéine(s) KGF-2 est/sont modifiée(s) chimiquement par conjugaison avec un polymère hydrosoluble.

7. Méthode selon l'une quelconque des revendications 1 à 6, dans laquelle, avant administration, ladite/lesdites protéine(s) KGF-2 est/sont reconstituées à partir d'une poudre déshydratée ou lyophilisée.

8. Utilisation d'une quantité efficace d'une/de protéine(s) KGF-2 pour la production d'un médicament destiné à la prophylaxie ou au traitement d'un état chez un patient souffrant d'un état choisi parmi les ulcères gastriques, les ulcères duodénaux, les érosions de l'estomac et de l'oesophage, la gastrite érosive, l'oesophagite, ou le reflux oesophagien, la maladie intestinale inflammatoire, la toxicité gastrointestinale induite par chimiothérapie ou par radiation, la cirrhose hépatique, l'insuffisance hépatique fulminante, l'hépatite virale aiguë, l'agression toxique du foie, et les diabètes.

9. Utilisation selon la revendication 8, dans laquelle ladite/lesdites protéine(s) KGF-2 a/ont une séquence d'acides aminés comprenant les acides aminés Cys³⁷ à Ser²⁰⁸ de SEQ ID n°2 ou une variante de séquence de celle-ci, dans laquelle ladite variante comprend des suppressions, des insertions, des substitutions et/ou des ajouts et retient l'activité biologique de la protéine KGF-2 mature.

10. Utilisation selon la revendication 11, dans laquelle ladite variante de séquence comprend une séquence d'acides aminés étant à plus de quatre-vingts pour cent (80 %), quatre-vingt-dix pour cent (90 %), quatre-vingt-quinze pour cent (95 %) ou quatre-vingt-dix-neuf pour cent (99 %) homologue aux acides aminés Cys³⁷ à Ser²⁰⁸ de SEQ ID n°2 et dans laquelle ladite variante comprend des suppressions, des insertions, des substitutions et/ou des ajouts et retient l'activité biologique de la protéine KGF-2 mature.

11. Utilisation selon la revendication 9, dans laquelle ladite/lesdites protéine(s) KGF-2 est/sont fusionnée(s) avec tout ou partie du domaine constant de la chaîne lourde ou légère de l'immunoglobuline humaine.

12. Utilisation selon l'une quelconque des revendications 8 à 11, dans laquelle ladite/lesdites protéine(s) KGF-2 est/sont modifiée(s) chimiquement par liaison à un polymère.

13. Utilisation selon la revendication 12, dans laquelle ledit/lesdites protéine(s) KGF-2 est/sont modifiée(s) chimiquement par conjugaison avec un polymère hydrosoluble.

14. Utilisation selon l'une quelconque des revendications 8 à 13, dans laquelle, avant administration, ladite/lesdites protéine(s) KGF-2 est/sont reconstituée(s) à partir d'une poudre déshydratée ou lyophilisée.

15. Utilisation selon l'une quelconque des revendications 8 à 14, dans laquelle ladite/lesdites protéine(s) KGF-2 est/sont administrée(s) via une voie topique, entérale ou parentérale.


### SEQUENCE LISTING

<110> Amgen Inc.
<120> USES OF KERATINOCYTE GROWTH FACTOR-2
<130> A-422C
<140> PCT/US97/18667
   <141> 1997-10-15
<150> 60/028,495
   <151> 1996-10-15
<150> 60/032,253
   <151> 1996-12-06
<150> 60/033,457
   <151> 1996-12-10
<160> 16
<170> Patent In Ver. 2.0
<210> 1
   <211> 627
   <212> DNA
   <213> Recombinant Human
<220>
   <221> CDS
   <222> (1)..(624)
<400> 1
atg tgg aaa tgg ata ctg aca cat tgt gcc tca gcc ttt ccc cac ctg     48
   ccc ggc tgc tgc tgc tgc tgc ttt ttg ttg ctg ttc ttg gtg tct tcc     96
   gtc cct gtc acc tgc caa gcc ctt ggt cag gac atg gtg tca cca gag    144
   gcc acc aac tct tct tcc tcc tcc ttc tcc tct cct tcc agc gcg gga    192
   agg cat gtg cgg agc tac aat cac ctt caa gga gat gtc cgc tgg aga    240
   aag cta ttc tct ttc acc aag tac ttt ctc aag att gag aag aac ggg    288
   aag gtc agc ggg acc aag aag gag aac tgc ccg tac agc atc ctg gag    336
   ata aca tca gta gaa atc gga gtt gtt gcc gtc aaa gcc att aac agc     384
   aac tat tac tta gcc atg aac aag aag ggg aaa ctc tat ggc tca aaa     432
   gaa ttt aac aat gac tgt aag ctg aag gag agg ata gag gaa aat gga     480
   tac aat acc tat gca tca ttt aac tgg cag cat aat ggg agg caa atg     528
   tat gtg gca ttg aat gga aaa gga gct cca agg aga gga cag aaa aca     576
   cga agg aaa aac acc tct gct cac ttt ctt cca atg gtg gta cac tca     624
   tag                                                                 627
<210> 2
   <211> 208
   <212> PRT
   <213> Recombinant Human
<400> 2
<210> 3
   <211> 588
   <212> DNA
   <213> Recombinant Human
<220>
   <221> CDS
   <222> (1)..(585)
<400> 3
   atg aaa cat cat cac cat cac cat cat gct agc gat gac gat gat aaa 48
   Met Lys His His His His His His His Ala Ser Asp Asp Asp Asp Lys
   1 5 10 15
   cag gct ctg ggt cag gac atg gtt tct ccg gag gct acc aac tcc tct 96
   Gln Ala Leu Gly Gln Asp Met Val Ser Pro Glu Ala Thr Asn Ser Ser
   20 25 30
   tcc tcc tcc tct tcc tcc tcc tcg tcc tct tcc ttc tcc tct cct tcc 144
   Ser Ser Ser Ser Ser Ser Ser Ser Ser Ser Ser Phe Ser Ser Pro Ser
   35 40 45
   agc gcg ggg agg cat gtg cga agc tac aat cac ctc cag gga gat gtc 192
   Ser Ala Gly Arg His Val Arg Ser Tyr Asn His Leu Gln Gly Asp Val
   50 55 60
   cgc tgg aga aag ctg ttc tcc ttc acc aag tac ttt ctc aag att gaa 240
   Arg Trp Arg Lys Leu Phe Ser Phe Thr Lys Tyr Phe Leu Lys Ile Glu
   65 70 75 80
   aag aac ggc aag gtc agc ggg acc aag aag gaa aac tgt ccg tac agt 288
   Lys Asn Gly Lys Val Ser Gly Thr Lys Lys Glu Asn Cys Pro Tyr Ser
   85 90 95
   atc cta gag ata aca tca gtg gaa atc gga gtt gtt gcc gtc aaa gcc 336
   Ile Leu Glu Ile Thr Ser Val Glu Ile Gly Val Val Ala Val Lys Ala
   100 105 110
   att aac agc aac tat tac tta gcc atg aac aag aag ggg aaa ctc tat 384
   Ile Asn Ser Asn Tyr Tyr Leu Ala Met Asn Lys Lys Gly Lys Leu Tyr
   115 120 125
   ggc tca aaa gaa ttt aac aat gac tgt aaa ctg aaa gag agg ata gag 432
   Gly Ser Lys Glu Phe Asn Asn Asp Cys Lys Leu Lys Glu Arg Ile Glu
   130 135 140
   gaa aat gga tac aac acc tat gca tct ttt aac tgg cag cac aac ggc 480
   Glu Asn Gly Tyr Asn Thr Tyr Ala Ser Phe Asn Trp Gln His Asn Gly
   145 150 155 160
   agg caa atg tat gtg gca ttg aat gga aaa gga gct ccc agg aga gga 528
   Arg Gln Met Tyr Val Ala Leu Asn Gly Lys Gly Ala Pro Arg Arg Gly
   165 170 175
   caa aaa aca aga agg aaa aac acc tcc gct cac ttc ctc ccc atg gtg 576
   Gln Lys Thr Arg Arg Lys Asn Thr Ser Ala His Phe Leu Pro Met Val
   180 185 190
   gtc cac tca taa 588
   Val His Ser
   195
<210> 4
   <211> 195
   <212> PRT
   <213> Recombinant Human
<400> 4
   Met Lys His His His His His His His Ala Ser Asp Asp Asp Asp Lys
   1               5                   10                  15
   Gln Ala Leu Gly Gln Asp Met Val Ser Pro Glu Ala Thr Asn Ser Ser
               20                  25                  30
   Ser Ser Ser Ser Ser Ser Ser Ser Ser Ser Ser Phe Ser Ser Pro Ser
           35                  40                  45
   Ser Ala Gly Arg His Val Arg Ser Tyr Asn His Leu Gln Gly Asp Val
       50                  55                  60
   Arg Trp Arg Lys Leu Phe Ser Phe Thr Lys Tyr Phe Leu Lys Ile Glu
   65                  70                  75                  80
   Lys Asn Gly Lys Val Ser Gly Thr Lys Lys Glu Asn Cys Pro Tyr Ser
                   85                  90                  95
   Ile Leu Glu Ile Thr Ser Val Glu Ile Gly Val Val Ala Val Lys Ala
               100                 105                 110
   Ile Asn Ser Asn Tyr Tyr Leu Ala Met Asn Lys Lys Gly Lys Leu Tyr
           115                 120                 125
   Gly Ser Lys Glu Phe Asn Asn Asp Cys Lys Leu Lys Glu Arg Ile Glu
       130                 135                 140
   Glu Asn Gly Tyr Asn Thr Tyr Ala Ser Phe Asn Trp Gln His Asn Gly
   145                 150                 155                 160
   Arg Gln Met Tyr Val Ala Leu Asn Gly Lys Gly Ala Pro Arg Arg Gly
                   165                 170                 175
   Gln Lys Thr Arg Arg Lys Asn Thr Ser Ala His Phe Leu Pro Met Val
               180                 185                 190
   Val His Ser
           195
<210> 5
   <211> 513
   <212> DNA
   <213> Recombinant Human
<220>
   <221> CDS
   <222> (1)..(510)
<400> 5
   atg ctg ggt cag gac atg gtt tct ccg gag gct acc aac tct agc tcc    48
   Met Leu Gly Gln Asp Met Val Ser Pro Glu Ala Thr Asn Ser Ser Ser
     1               5                  10                  15
   agc agc ttc tcc tct cct agc tct gca ggt agg cat gtg cgg agc tac    96
   Ser Ser Phe Ser Ser Pro Ser Ser Ala Gly Arg His Val Arg Ser Tyr
                20                  25                  30
   aat cac ctc cag gga gat gtc cgc tgg aga aag ctg ttc tcc ttc acc   144
   Asn His Leu Gln Gly Asp Val Arg Trp Arg Lys Leu Phe Ser Phe Thr
            35                  40                  45
   aag tac ttt ctc aag att gaa aag aac ggc aag gtc agc ggg acc aag   192
   Lys Tyr Phe Leu Lys Ile Glu Lys Asn Gly Lys Val Ser Gly Thr Lys
        50                  55                  60
   aag gaa aac tgt ccg tac agt atc cta gag ata aca tca gtg gaa atc   240
   Lys Glu Asn Cys Pro Tyr Ser Ile Leu Glu Ile Thr Ser Val Glu Ile
    65                  70                  75                  80
   gga gtt gtt gcc gtc aaa gcc att aac agc aac tat tac tta gcc atg   288
   Gly Val Val Ala Val Lys Ala Ile Asn Ser Asn Tyr Tyr Leu Ala Met
                    85                  90                  95
   aac aag aag ggg aaa ctc tat ggc tca aaa gaa ttt aac aat gac tgt   336
   Asn Lys Lys Gly Lys Leu Tyr Gly Ser Lys Glu Phe Asn Asn Asp Cys
               100                 105                 110
   aaa ctg aaa gag agg ata gag gaa aat gga tac aac acc tat gca tct   384
   Lys Leu Lys Glu Arg Ile Glu Glu Asn Gly Tyr Asn Thr Tyr Ala Ser
           115                 120                 125
   ttt aac tgg cag cac aac ggc agg caa atg tat gtg gca ttg aat gga   432
   Phe Asn Trp Gln His Asn Gly Arg Gln Met Tyr Val Ala Leu Asn Gly
       130                 135                 140
   aaa gga gct ccc agg aga gga caa aaa aca aga agg aaa aac acc tcc   480
   Lys Gly Ala Pro Arg Arg Gly Gln Lys Thr Arg Arg Lys Asn Thr Ser
   145                 150                 155                 160
   gct cac ttc ctc ccc atg gtg gtc cac tca taa                       513
   Ala His Phe Leu Pro Met Val Val His Ser
                   165                 170
<210> 6
   <211> 170
   <212> PRT
   <213> Recombinant Human
<400> 6
   Met Leu Gly Gln Asp Met Val Ser Pro Glu Ala Thr Asn Ser Ser Ser
   1               5                   10                  15
   Ser Ser Phe Ser Ser Pro Ser Ser Ala Gly Arg His Val Arg Ser Tyr
               20                  25                  30
   Asn His Leu Gln Gly Asp Val Arg Trp Arg Lys Leu Phe Ser Phe Thr
           35                  40                  45
   Lys Tyr Phe Leu Lys Ile Glu Lys Asn Gly Lys Val Ser Gly Thr Lys
       50                  55                  60
   Lys Glu Asn Cys Pro Tyr Ser Ile Leu Glu Ile Thr Ser Val Glu Ile
   65                  70                  75                  80
   Gly Val Val Ala Val Lys Ala Ile Asn Ser Asn Tyr Tyr Leu Ala Met
                   85                  90                  95
   Asn Lys Lys Gly Lys Leu Tyr Gly Ser Lys Glu Phe Asn Asn Asp Cys
               100                 105                 110
   Lys Leu Lys Glu Arg Ile Glu Glu Asn Gly Tyr Asn Thr Tyr Ala Ser
           115                 120                 125
   Phe Asn Trp Gln His Asn Gly Arg Gln Met Tyr Val Ala Leu Asn Gly
       130                 135                 140
   Lys Gly Ala Pro Arg Arg Gly Gln Lys Thr Arg Arg Lys Asn Thr Ser
   145                 150                 155                 160
   Ala His Phe Leu Pro Met Val Val His Ser
                   165                 170
<210> 7
   <211> 36
   <212> DNA
   <213> Recombinant Human
<400> 7
   aaacaacata tggtttctcc ggaggctacc aactcc 36
<210> 8
   <211> 35
   <212> DNA
   <213> Recombinant Human
<400> 8
   aaacaaggat cctttatgag tggaccacca tgggg. 35
<210> 9
   <211> 47
   <212> DNA
   <213> Recombinant Human
<400> 9
   ctagcgatga cgatgataaa caggctctgg gtcaggacat ggtttct 47
<210> 10
   <211> 47
   <212> DNA
   <213> Recombinant Human
<400> 10
   ccggagaaac catgtcctga cccagagcct gtttatcatc gtcatcg 47
<210> 11
   <211> 53
   <212> DNA
   <213> Recombinant Human
<400> 11
   aaacaacata tgtcttctcc ttcctctgca ggtaggcatg tgcggagcta caa 53
<210> 12
   <211> 35
   <212> DNA
   <213> Recombinant Human
<400> 12
   aaacaaggat cctttatgag tggaccacca tgggg 35
<210> 13
   <211> 25
   <212> DNA
   <213> Recombinant Human
<400> 13
   tatgctgggt caggacatgg tttct 25
<210> 14
   <211> 27
   <212> DNA
   <213> Recombinant Human
<400> 14
   ccggagaaac catgtcctga cccagca 27
<210> 15
   <211> 51
   <212> DNA
   <213> Recombinant Human
<400> 15
   ccggaggcta ccaactctag ctccagcagc ttctcptctc ctagctctgc a 51
<210> 16
   <211> 43
   <212> DNA
   <213> Recombinant Human
<400> 16
   gagctaggag aggagaagct gctggagcta gagttggtag cct 43

### SEQUENCE LISTING

<110> Amgen Inc.
<120> USES OF KERATINOCYTE GROWTH FACTOR-2
<130> A-422C
<140> PCT/US97/18667
   <141> 1997-10-15
<150> 60/028,495
   <151> 1996-10-15
<150> 60/032,253
   <151> 1996-12-06
<150> 60/033,457
   <151> 1996-12-10
<160> 16
<170> PatentIn Ver. 2.0
<210> 1
   <211> 627
   <212> DNA
   <213> Recombinant Human
<220>
   <221> CDS
   <222> (1)..(624)
<400> 1
atg tgg aaa tgg ata ctg aca cat tgt gcc tca gcc ttt ccc cac ctg 48
   ccc ggc tgc tgc tgc tgc tgc ttt ttg ttg ctg ttc ttg gtg tct tcc 96
   gtc cct gtc acc tgc caa gcc ctt ggt cag gac atg gtg tca cca gag 144
   gcc acc aac tct tct tcc tcc tcc ttc tcc tct cct tcc agc gcg gga 192
   agg cat gtg cgg agc tac aat cac ctt caa gga gat gtc cgc tgg aga 240
   aag cta ttc tct ttc acc aag tac ttt ctc aag att gag aag aac ggg 288
   aag gtc agc ggg acc aag aag gag aac tgc ccg tac agc atc ctg gag 336
   ata aca tca gta gaa atc gga gtt gtt gcc gtc aaa gcc att aac agc 384
   aac tat tac tta gcc atg aac aag aag ggg aaa ctc tat ggc tca aaa 432
   gaa ttt aac aat gac tgt aag ctg aag gag agg ata gag gaa aat gga 480
   tac aat acc tat gca tca ttt aac tgg cag cat aat ggg agg caa atg 528
   tat gtg gca ttg aat gga aaa gga gct cca agg aga gga cag aaa aca 576
   cga agg aaa aac acc tct gct cac ttt ctt cca atg gtg gta cac tca 624
   tag 627
<210> 2
   <211> 208
   <212> PRT
   <213> Recombinant Human
<400> 2
<210> 3
   <211> 588
   <212> DNA
   <213> Recombinant Human
<220>
   <221> CDS
   <222> (1)..(585)
<400> 3
   atg aaa cat cat cac cat cac cat cat gct agc gat gac gat gat aaa    48
   Met Lys His His His His His His His Ala Ser Asp Asp Asp Asp Lys
   1               5                   10                  15
   cag gct ctg ggt cag gac atg gtt tct ccg gag gct acc aac tcc tct    96
   Gln Ala Leu Gly Gln Asp Met Val Ser Pro Glu Ala Thr Asn Ser Ser
               20                  25                  30
   tcc tcc tcc tct tcc tcc tcc tcg tcc tct tcc ttc tcc tct cct tcc   144
   Ser Ser Ser Ser Ser Ser Ser Ser Ser Ser Ser Phe Ser Ser Pro Ser
           35                  40                  45
   agc gcg ggg agg cat gtg cga agc tac aat cac ctc cag gga gat gtc   192
   Ser Ala Gly Arg His Val Arg Ser Tyr Asn His Leu Gln Gly Asp Val
       50                  55                  60
   cgc tgg aga aag ctg ttc tcc ttc acc aag tac ttt ctc aag att gaa   240
   Arg Trp Arg Lys Leu Phe Ser Phe Thr Lys Tyr Phe Leu Lys Ile Glu
   65                  70                  75                  80
   aag aac ggc aag gtc agc ggg acc aag aag gaa aac tgt ccg tac agt   288
   Lys Asn Gly Lys Val Ser Gly Thr Lys Lys Glu Asn Cys Pro Tyr Ser
                   85                  90                  95
   atc cta gag ata aca tca gtg gaa atc gga gtt gtt gcc gtc aaa gcc   336
   Ile Leu Glu Ile Thr Ser Val Glu Ile Gly Val Val Ala Val Lys Ala
               100                 105                 110
   att aac agc aac tat tac tta gcc atg aac aag aag ggg aaa ctc tat   384
   Ile Asn Ser Asn Tyr Tyr Leu Ala Met Asn Lys Lys Gly Lys Leu Tyr
           115                 120                 125
   ggc tca aaa gaa ttt aac aat gac tgt aaa ctg aaa gag agg ata gag   432
   Gly Ser Lys Glu Phe Asn Asn Asp Cys Lys Leu Lys Glu Arg Ile Glu
       130                 135                 140
   gaa aat gga tac aac acc tat gca tct ttt aac tgg cag cac aac ggc   480
   Glu Asn Gly Tyr Asn Thr Tyr Ala Ser Phe Asn Trp Gln His Asn Gly
   145                 150                 155                 160
   agg caa atg tat gtg gca ttg aat gga aaa gga gct ccc agg aga gga   528
   Arg Gln Met Tyr Val Ala Leu Asn Gly Lys Gly Ala Pro Arg Arg Gly
                   165                 170                 175
   caa aaa aca aga agg aaa aac acc tcc gct cac ttc ctc ccc atg gtg   576
   Gln Lys Thr Arg Arg Lys Asn Thr Ser Ala His Phe Leu Pro Met Val
               180                 185                 190
   gtc cac tca taa                                                   588
   Val His Ser
           195
<210> 4
   <211> 195
   <212> PRT
   <213> Recombinant Human
<400> 4
   Met Lys His His His His His His His Ala Ser Asp Asp Asp Asp Lys
   1               5                   10                  15
   Gln Ala Leu Gly Gln Asp Met Val Ser Pro Glu Ala Thr Asn Ser Ser
               20                  25                  30
   Ser Ser Ser Ser Ser Ser Ser Ser Ser Ser Ser Phe Ser Ser Pro Ser
           35                  40                  45
   Ser Ala Gly Arg His Val Arg Ser Tyr Asn His Leu Gln Gly Asp Val
       50                  55                  60
   Arg Trp Arg Lys Leu Phe Ser Phe Thr Lys Tyr Phe Leu Lys Ile Glu
   65                  70                  75                  80
   Lys Asn Gly Lys Val Ser Gly Thr Lys Lys Glu Asn Cys Pro Tyr Ser
                   85                  90                  95
   Ile Leu Glu Ile Thr Ser Val Glu Ile Gly Val Val Ala Val Lys Ala
               100                 105                 110
   Ile Asn Ser Asn Tyr Tyr Leu Ala Met Asn Lys Lys Gly Lys Leu Tyr
           115                 120                 125
   Gly Ser Lys Glu Phe Asn Asn Asp Cys Lys Leu Lys Glu Arg Ile Glu
       130                 135                 140
   Glu Asn Gly Tyr Asn Thr Tyr Ala Ser Phe Asn Trp Gln His Asn Gly
   145                 150                 155                 160
   Arg Gln Met Tyr Val Ala Leu Asn Gly Lys Gly Ala Pro Arg Arg Gly
                   165                 170                 175
   Gln Lys Thr Arg Arg Lys Asn Thr Ser Ala His Phe Leu Pro Met Val
               180                 185                 190
   Val His Ser
           195
<210> 5
   <211> 513
   <212> DNA
   <213> Recombinant Human
<220>
   <221> CDS
   <222> (1)..(510)
<400> 5
   atg ctg ggt cag gac atg gtt tct ccg gag gct acc aac tct agc tcc      48
   Met Leu Gly Gln Asp Met Val Ser Pro Glu Ala Thr Asn Ser Ser Ser
    1               5                  10                  15
   agc agc ttc tcc tct cct agc tct gca ggt agg cat gtg cgg agc tac      96
   Ser Ser Phe Ser Ser Pro Ser Ser Ala Gly Arg His Val Arg Ser Tyr
               20                  25                  30
   aat cac ctc cag gga gat gtc cgc tgg aga aag ctg ttc tcc ttc acc     144
   Asn His Leu Gln Gly Asp Val Arg Trp Arg Lys Leu Phe Ser Phe Thr
           35                  40                  45
   aag tac ttt ctc aag att gaa aag aac ggc aag gtc agc ggg acc aag     192
   Lys Tyr Phe Leu Lys Ile Glu Lys Asn Gly Lys Val Ser Gly Thr Lys
       50                  55                  60
   aag gaa aac tgt ccg tac agt atc cta gag ata aca tca gtg gaa atc     240
   Lys Glu Asn Cys Pro Tyr Ser Ile Leu Glu Ile Thr Ser Val Glu Ile
   65                  70                  75                  80
   gga gtt gtt gcc gtc aaa gcc att aac agc aac tat tac tta gcc atg     288
   Gly Val Val Ala Val Lys Ala Ile Asn Ser Asn Tyr Tyr Leu Ala Met
                   85                  90                  95
   aac aag aag ggg aaa ctc tat ggc tca aaa gaa ttt aac aat gac tgt     336
   Asn Lys Lys Gly Lys Leu Tyr Gly Ser Lys Glu Phe Asn Asn Asp Cys
               100                 105                 110
   aaa ctg aaa gag agg ata gag gaa aat gga tac aac acc tat gca tct     384
   Lys Leu Lys Glu Arg Ile Glu Glu Asn Gly Tyr Asn Thr Tyr Ala Ser
           115                 120                 125
   ttt aac tgg cag cac aac ggc agg caa atg tat gtg gca ttg aat gga     432
   Phe Asn Trp Gln His Asn Gly Arg Gln Met Tyr Val Ala Leu Asn Gly
       130                 135                 140
   aaa gga gct ccc agg aga gga caa aaa aca aga agg aaa aac acc tcc     480
   Lys Gly Ala Pro Arg Arg Gly Gln Lys Thr Arg Arg Lys Asn Thr Ser
   145                 150                 155                 160
   gct cac ttc ctc ccc atg gtg gtc cac tca taa                         513
   Ala His Phe Leu Pro Met Val Val His Ser
                   165                 170
<210> 6
   <211> 170
   <212> PRT
   <213> Recombinant Human
<400> 6
   Met Leu Gly Gln Asp Met Val Ser Pro Glu Ala Thr Asn Ser Ser Ser
    1               5                  10                  15
   Ser Ser Phe Ser Ser Pro Ser Ser Ala Gly Arg His Val Arg Ser Tyr
               20                  25                  30
   Asn His Leu Gln Gly Asp Val Arg Trp Arg Lys Leu Phe Ser Phe Thr
           35                  40                  45
   Lys Tyr Phe Leu Lys Ile Glu Lys Asn Gly Lys Val Ser Gly Thr Lys
       50                  55                  60
   Lys Glu Asn Cys Pro Tyr Ser Ile Leu Glu Ile Thr Ser Val Glu Ile
   65                  70                  75                  80
   Gly Val Val Ala Val Lys Ala Ile Asn Ser Asn Tyr Tyr Leu Ala Met
                   85                  90                  95
   Asn Lys Lys Gly Lys Leu Tyr Gly Ser Lys Glu Phe Asn Asn Asp Cys
               100                 105                 110
   Lys Leu Lys Glu Arg Ile Glu Glu Asn Gly Tyr Asn Thr Tyr Ala Ser
           115                 120                 125
   Phe Asn Trp Gln His Asn Gly Arg Gln Met Tyr Val Ala Leu Asn Gly
       130                 135                 140
   Lys Gly Ala Pro Arg Arg Gly Gln Lys Thr Arg Arg Lys Asn Thr Ser
   145                 150                 155                 160
   Ala His Phe Leu Pro Met Val Val His Ser
                   165                 170
<210> 7
   <211> 36
   <212> DNA
   <213> Recombinant Human
<400> 7
   aaacaacata tggtttctcc ggaggctacc aactcc 36
<210> 8
   <211> 35
   <212> DNA
   <213> Recombinant Human
<400> 8
   aaacaaggat cctttatgag tggaccacca tgggg 35
<210> 9
   <211> 47
   <212> DNA
   <213> Recombinant Human
<400> 9
   ctagcgatga cgatgataaa caggctctgg gtcaggacat ggtttct 47
<210> 10
   <211> 47
   <212> DNA
   <213> Recombinant Human
<400> 10
   ccggagaaac catgtcctga cccagagcct gtttatcatc gtcatcg 47
<210> 11
   <211> 53
   <212> DNA
   <213> Recombinant Human
<400> 11
   aaacaacata tgtcttctcc ttcctctgca ggtaggcatg tgcggagcta caa 53
<210> 12
   <211> 35
   <212> DNA
   <213> Recombinant Human
<400> 12
   aaacaaggat cctttatgag tggaccacca tgggg 35
<210> 13
   <211> 25
   <212> DNA
   <213> Recombinant Human
<400> 13
   tatgctgggt caggacatgg tttct 25
<210> 14
   <211> 27
   <212> DNA
   <213> Recombinant Human
<400> 14
   ccggagaaac catgtcctga cccagca 27
<210> 15
   <211> 51
   <212> DNA
   <213> Recombinant Human
<400> 15
   ccggaggcta ccaactctag ctccagcagc ttctcctctc ctagctctgc a 51
<210> 16
   <211> 43
   <212> DNA
   <213> Recombinant Human
<400> 16
   gagctaggag aggagaagct gctggagcta gagttggtag cct 43

### SEQUENCE LISTING

(1) GENERAL INFORMATION:
   (i) APPLICANT: Amgen Inc.
   (ii) TITLE OF INVENTION: USES OF KERATINOCYTE GROWTH FACTOR-2
   (iii) NUMBER OF SEQUENCES: 16
   (iv) CORRESPONDENCE ADDRESS:
      (A) ADDRESSEE: Amgen Inc.
      (B) STREET: 1840 De Havilland Drive
      (C) CITY: Thousand Oaks
      (D) STATE: CA
      (E) COUNTRY: USA
      (F) ZIP: 91320-1789
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30
   (vi) CURRENT APPLICATION DATA:
      (A) APPLICATION NUMBER:
      (B) FILING DATE:
      (C) CLASSIFICATION:
   (vii) PRIOR APPLICATION DATA:
      (A) APPLICATION NUMBER: US 60/028,495
      (B) FILING DATE: 15-OCT-1996
   (vii) PRIOR APPLICATION DATA:
      (A) APPLICATION NUMBER: US 60/032,253
      (B) FILING DATE: 06-DEC-1996
   (vii) PRIOR APPLICATION DATA:
      (A) APPLICATION NUMBER: US 60/033,457
      (B) FILING DATE: 10-DEC-1996
(2) INFORMATION FOR SEQ ID NO:1:
   (i) SEQUENCE CHARACTERISTICS:
      (A) LENGTH: 627 base pairs
      (B) TYPE: nucleic acid
      (C) STRANDEDNESS: unknown
      (D) TOPOLOGY: unknown
   (ii) MOLECULE TYPE: cDNA
   (ix) FEATURE:
      (A) NAME/KEY: CDS
      (B) LOCATION: 1..627
   (xi) SEQUENCE DESCRIPTION: SEQ ID NO:1:
ATG TGG AAA TGG ATA CTG ACA CAT TGT GCC TCA GCC TTT CCC CAC CTG      48
   CCC GGC TGC TGC TGC TGC TGC TTT TTG TTG CTG TTC TTG GTG TCT TCC      96
   GTC CCT GTC ACC TGC CAA GCC CTT GGT CAG GAC ATG GTG TCA CCA GAG     144
   GCC ACC AAC TCT TCT TCC TCC TCC TTC TCC TCT CCT TCC AGC GCG GGA     192
   AGG CAT GTG CGG AGC TAC AAT CAC CTT CAA GGA GAT GTC CGC TGG AGA     240
   AAG CTA TTC TCT TTC ACC AAG TAC TTT CTC AAG ATT GAG AAG AAC GGG     288
   AAG GTC AGC GGG ACC AAG AAG GAG AAC TGC CCG TAC AGC ATC CTG GAG     336
   ATA ACA TCA GTA GAA ATC GGA GTT GTT GCC GTC AAA GCC ATT AAC AGC     384
   AAC TAT TAC TTA GCC ATG AAC AAG AAG GGG AAA CTC TAT GGC TCA AAA     432
   GAA TTT AAC AAT GAC TGT AAG CTG AAG GAG AGG ATA GAG GAA AAT GGA     480
   TAC AAT ACC TAT GCA TCA TTT AAC TGG CAG CAT AAT GGG AGG CAA ATG     528
   TAT GTG GCA TTG AAT GGA AAA GGA GCT CCA AGG AGA GGA CAG AAA ACA     576
   CGA AGG AAA AAC ACC TCT GCT CAC TTT CTT CCA ATG GTG GTA CAC TCA     624
   TAG                                                                 627
(2) INFORMATION FOR SEQ ID NO:2:
   (i) SEQUENCE CHARACTERISTICS:
      (A) LENGTH: 209 amino acids
      (B) TYPE: amino acid
      (D) TOPOLOGY: unknown
   (ii) MOLECULE TYPE: protein
   (xi) SEQUENCE DESCRIPTION: SEQ ID NO:2:
(2) INFORMATION FOR SEQ ID NO:3:
   (i) SEQUENCE CHARACTERISTICS:
      (A) LENGTH: 588 base pairs
      (B) TYPE: nucleic acid
      (C) STRANDEDNESS: unknown
      (D) TOPOLOGY: unknown
   (ii) MOLECULE TYPE: cDNA
   (ix) FEATURE:
      (A) NAME/KEY: CDS
      (B) LOCATION: 1..588
   (xi) SEQUENCE DESCRIPTION: SEQ ID NO:3:
   ATG AAA CAT CAT CAC CAT CAC CAT CAT GCT AGC GAT GAC GAT GAT AAA      48
   Met Lys His His His His His His His Ala Ser Asp Asp Asp Asp Lys
    1               5                  10                  15
   CAG GCT CTG GGT CAG GAC ATG GTT TCT CCG GAG GCT ACC AAC TCC TCT      96
   Gln Ala Leu Gly Gln Asp Met Val Ser Pro Glu Ala Thr Asn Ser Ser
               20                  25                  30
   TCC TCC TCC TCT TCC TCC TCC TCG TCC TCT TCC TTC TCC TCT CCT TCC     144
   Ser Ser Ser Ser Ser Ser Ser Ser Ser Ser Ser Phe Ser Ser Pro Ser
           35                  40                  45
   AGC GCG GGG AGG CAT GTG CGA AGC TAC AAT CAC CTC CAG GGA GAT GTC     192
   Ser Ala Gly Arg His Val Arg Ser Tyr Asn His Leu Gln Gly Asp Val
       50                  55                  60
   CGC TGG AGA AAG CTG TTC TCC TTC ACC AAG TAC TTT CTC AAG ATT GAA     240
   Arg Trp Arg Lys Leu Phe Ser Phe Thr Lys Tyr Phe Leu Lys Ile Glu
   65                  70                  75                  80
   AAG AAC GGC AAG GTC AGC GGG ACC AAG AAG GAA AAC TGT CCG TAC AGT     288
   Lys Asn Gly Lys Val Ser Gly Thr Lys Lys Glu Asn Cys Pro Tyr Ser
                   85                  90                  95
   ATC CTA GAG ATA ACA TCA GTG GAA ATC GGA GTT GTT GCC GTC AAA GCC     336
   Ile Leu Glu Ile Thr Ser Val Glu Ile Gly Val Val Ala Val Lys Ala
               100                 105                 110
   ATT AAC AGC AAC TAT TAC TTA GCC ATG AAC AAG AAG GGG AAA CTC TAT     384
   Ile Asn Ser Asn Tyr Tyr Leu Ala Met Asn Lys Lys Gly Lys Leu Tyr
           115                 120                 125
   GGC TCA AAA GAA TTT AAC AAT GAC TGT AAA CTG AAA GAG AGG ATA GAG     432
   Gly Ser Lys Glu Phe Asn Asn Asp Cys Lys Leu Lys Glu Arg Ile Glu
       130                 135                 140
   GAA AAT GGA TAC AAC ACC TAT GCA TCT TTT AAC TGG CAG CAC AAC GGC     480
   Glu Asn Gly Tyr Asn Thr Tyr Ala Ser Phe Asn Trp Gln His Asn Gly
   145                 150                 155                 160
   AGG CAA ATG TAT GTG GCA TTG AAT GGA AAA GGA GCT CCC AGG AGA GGA     528
   Arg Gln Met Tyr Val Ala Leu Asn Gly Lys Gly Ala Pro Arg Arg Gly
                   165                 170                 175
   CAA AAA ACA AGA AGG AAA AAC ACC TCC GCT CAC TTC CTC CCC ATG GTG     576
   Gln Lys Thr Arg Arg Lys Asn Thr Ser Ala His Phe Leu Pro Met Val
               180                 185                 190
   GTC CAC TCA TAA                                                     588
   Val His Ser  *
           195
(2) INFORMATION FOR SEQ ID NO:4:
   (i) SEQUENCE CHARACTERISTICS:
      (A) LENGTH: 196 amino acids
      (B) TYPE: amino acid
      (D) TOPOLOGY: unknown
   (ii) MOLECULE TYPE: protein
   (xi) SEQUENCE DESCRIPTION: SEQ ID NO:4:
   Met Lys His His His His His His His Ala Ser Asp Asp Asp Asp Lys
    1               5                  10                  15
   Gln Ala Leu Gly Gln Asp Met Val Ser Pro Glu Ala Thr Asn Ser Ser
               20                  25                  30
   Ser Ser Ser Ser Ser Ser Ser Ser Ser Ser Ser Phe Ser Ser Pro Ser
           35                  40                  45
   Ser Ala Gly Arg His Val Arg Ser Tyr Asn His Leu Gln Gly Asp Val
       50                  55                  60
   Arg Trp Arg Lys Leu Phe Ser Phe Thr Lys Tyr Phe Leu Lys Ile Glu
   65                  70                  75                  80
   Lys Asn Gly Lys Val Ser Gly Thr Lys Lys Glu Asn Cys Pro Tyr Ser
                   85                  90                  95
   Ile Leu Glu Ile Thr Ser Val Glu Ile Gly Val Val Ala Val Lys Ala
               100                 105                 110
   Ile Asn Ser Asn Tyr Tyr Leu Ala Met Asn Lys Lys Gly Lys Leu Tyr
           115                 120                 125
   Gly Ser Lys Glu Phe Asn Asn Asp Cys Lys Leu Lys Glu Arg Ile Glu
       130                 135                 140
   Glu Asn Gly Tyr Asn Thr Tyr Ala Ser Phe Asn Trp Gln His Asn Gly
   145                 150                 155                 160
   Arg Gln Met Tyr Val Ala Leu Asn Gly Lys Gly Ala Pro Arg Arg Gly
                   165                 170                 175
   Gln Lys Thr Arg Arg Lys Asn Thr Ser Ala His Phe Leu Pro Met Val
               180                 185                 190
   Val His Ser  *
           195
(2) INFORMATION FOR SEQ ID NO:5:
   (i) SEQUENCE CHARACTERISTICS:
      (A) LENGTH: 513 base pairs
      (B) TYPE: nucleic acid
      (C) STRANDEDNESS: unknown
      (D) TOPOLOGY: unknown
   (ii) MOLECULE TYPE: cDNA
   (ix) FEATURE:
      (A) NAME/KEY: CDS
      (B) LOCATION: 1..513
   (xi) SEQUENCE DESCRIPTION: SEQ ID NO:5:
   ATG CTG GGT CAG GAC ATG GTT TCT CCG GAG GCT ACC AAC TCT AGC TCC     48
   Met Leu Gly Gln Asp Met Val Ser Pro Glu Ala Thr Asn Ser Ser Ser
    1               5                  10                  15
   AGC AGC TTC TCC TCT CCT AGC TCT GCA GGT AGG CAT GTG CGG AGC TAC     96
   Ser Ser Phe Ser Ser Pro Ser Ser Ala Gly Arg His Val Arg Ser Tyr
                20                  25                  30
   AAT CAC CTC CAG GGA GAT GTC CGC TGG AGA AAG CTG TTC TCC TTC ACC    144
   Asn His Leu Gln Gly Asp Val Arg Trp Arg Lys Leu Phe Ser Phe Thr
            35                  40                  45
   AAG TAC TTT CTC AAG ATT GAA AAG AAC GGC AAG GTC AGC GGG ACC AAG    192
   Lys Tyr Phe Leu Lys Ile Glu Lys Asn Gly Lys Val Ser Gly Thr Lys
        50                  55                  60
   AAG GAA AAC TGT CCG TAC AGT ATC CTA GAG ATA ACA TCA GTG GAA ATC    240
   Lys Glu Asn Cys Pro Tyr Ser Ile Leu Glu Ile Thr Ser Val Glu Ile
    65                  70                  75                  80
   GGA GTT GTT GCC GTC AAA GCC ATT AAC AGC AAC TAT TAC TTA GCC ATG    288
   Gly Val Val Ala Val Lys Ala Ile Asn Ser Asn Tyr Tyr Leu Ala Met
                    85                  90                  95
   AAC AAG AAG GGG AAA CTC TAT GGC TCA AAA GAA TTT AAC AAT GAC TGT    336
   Asn Lys Lys Gly Lys Leu Tyr Gly Ser Lys Glu Phe Asn Asn Asp Cys
               100                 105                 110
   AAA CTG AAA GAG AGG ATA GAG GAA AAT GGA TAC AAC ACC TAT GCA TCT    384
   Lys Leu Lys Glu Arg Ile Glu Glu Asn Gly Tyr Asn Thr Tyr Ala Ser
           115                 120                 125
   TTT AAC TGG CAG CAC AAC GGC AGG CAA ATG TAT GTG GCA TTG AAT GGA    432
   Phe Asn Trp Gln His Asn Gly Arg Gln Met Tyr Val Ala Leu Asn Gly
       130                 135                 140
   AAA GGA GCT CCC AGG AGA GGA CAA AAA ACA AGA AGG AAA AAC ACC TCC    480
   Lys Gly Ala Pro Arg Arg Gly Gln Lys Thr Arg Arg Lys Asn Thr Ser
   145                 150                 155                 160
   GCT CAC TTC CTC CCC ATG GTG GTC CAC TCA TAA                        513
   Ala His Phe Leu Pro Met Val Val His Ser  *
                   165                 170
(2) INFORMATION FOR SEQ ID NO:6:
   (i) SEQUENCE CHARACTERISTICS:
      (A) LENGTH: 171 amino acids
      (B) TYPE: amino acid
      (D) TOPOLOGY: unknown
   (ii) MOLECULE TYPE: protein
   (xi) SEQUENCE DESCRIPTION: SEQ ID NO:6:
   Met Leu Gly Gln Asp Met Val Ser Pro Glu Ala Thr Asn Ser Ser Ser
    1               5                  10                  15
   Ser Ser Phe Ser Ser Pro Ser Ser Ala Gly Arg His Val Arg Ser Tyr
               20                  25                  30
   Asn His Leu Gln Gly Asp Val Arg Trp Arg Lys Leu Phe Ser Phe Thr
           35                  40                  45
   Lys Tyr Phe Leu Lys Ile Glu Lys Asn Gly Lys Val Ser Gly Thr Lys
       50                  55                  60
   Lys Glu Asn Cys Pro Tyr Ser Ile Leu Glu Ile Thr Ser Val Glu Ile
   65                  70                  75                  80
   Gly Val Val Ala Val Lys Ala Ile Asn Ser Asn Tyr Tyr Leu Ala Met
                   85                  90                  95
   Asn Lys Lys Gly Lys Leu Tyr Gly Ser Lys Glu Phe Asn Asn Asp Cys
               100                 105                 110
   Lys Leu Lys Glu Arg Ile Glu Glu Asn Gly Tyr Asn Thr Tyr Ala Ser
           115                 120                 125
   Phe Asn Trp Gln His Asn Gly Arg Gln Met Tyr Val Ala Leu Asn Gly
       130                 135                 140
   Lys Gly Ala Pro Arg Arg Gly Gln Lys Thr Arg Arg Lys Asn Thr Ser
   145                 150                 155                 160
   Ala His Phe Leu Pro Met Val Val His Ser  *
                   165                 170
(2) INFORMATION FOR SEQ ID NO:7:
   (i) SEQUENCE CHARACTERISTICS:
      (A) LENGTH: 36 base pairs
      (B) TYPE: nucleic acid
      (C) STRANDEDNESS: unknown
      (D) TOPOLOGY: unknown
   (ii) MOLECULE TYPE: cDNA
   (xi) SEQUENCE DESCRIPTION: SEQ ID NO:7:
      AAACAACATA TGGTTTCTCC GGAGGCTACC AACTCC 36
(2) INFORMATION FOR SEQ ID NO:8:
   (i) SEQUENCE CHARACTERISTICS:
      (A) LENGTH: 35 base pairs
      (B) TYPE: nucleic acid
      (C) STRANDEDNESS: unknown
      (D) TOPOLOGY: unknown
   (ii) MOLECULE TYPE: cDNA
   (xi) SEQUENCE DESCRIPTION: SEQ ID NO:8:
      AAACAAGGAT CCTTTATGAG TGGACCACCA TGGGG 35
(2) INFORMATION FOR SEQ ID NO:9:
   (i) SEQUENCE CHARACTERISTICS:
      (A) LENGTH: 47 base pairs
      (B) TYPE: nucleic acid
      (C) STRANDEDNESS: unknown
      (D) TOPOLOGY: unknown
   (ii) MOLECULE TYPE: cDNA
   (xi) SEQUENCE DESCRIPTION: SEQ ID NO:9:
      CTAGCGATGA CGATGATAAA CAGGCTCTGG GTCAGGACAT GGTTTCT 47
(2) INFORMATION FOR SEQ ID NO:10:
   (i) SEQUENCE CHARACTERISTICS:
      (A) LENGTH: 47 base pairs
      (B) TYPE: nucleic acid
      (C) STRANDEDNESS: unknown
      (D) TOPOLOGY: unknown
   (ii) MOLECULE TYPE: cDNA
   (xi) SEQUENCE DESCRIPTION: SEQ ID NO:10:
      CCGGAGAAAC CATGTCCTGA CCCAGAGCCT GTTTATCATC GTCATCG 47
(2) INFORMATION FOR SEQ ID NO:11:
   (i) SEQUENCE CHARACTERISTICS:
      (A) LENGTH: 53 base pairs
      (B) TYPE: nucleic acid
      (C) STRANDEDNESS: unknown
      (D) TOPOLOGY: unknown
   (ii) MOLECULE TYPE: cDNA
   (xi) SEQUENCE DESCRIPTION: SEQ ID NO:11:
      AAACAACATA TGTCTTCTCC TTCCTCTGCA GGTAGGCATG TGCGGAGCTA CAA 53
(2) INFORMATION FOR SEQ ID NO:12:
   (i) SEQUENCE CHARACTERISTICS:
      (A) LENGTH: 35 base pairs
      (B) TYPE: nucleic acid
      (C) STRANDEDNESS: unknown
      (D) TOPOLOGY: unknown
   (ii) MOLECULE TYPE: cDNA
   (xi) SEQUENCE DESCRIPTION: SEQ ID NO:12:
      AAACAAGGAT CCTTTATGAG TGGACCACCA TGGGG 35
(2) INFORMATION FOR SEQ ID NO:13:
   (i) SEQUENCE CHARACTERISTICS:
      (A) LENGTH: 25 base pairs
      (B) TYPE: nucleic acid
      (C) STRANDEDNESS: unknown
      (D) TOPOLOGY: unknown
   (ii) MOLECULE TYPE: cDNA
   (xi) SEQUENCE DESCRIPTION: SEQ ID NO:13:
      TATGCTGGGT CAGGACATGG TTTCT 25
(2) INFORMATION FOR SEQ ID NO:14:
   (i) SEQUENCE CHARACTERISTICS:
      (A) LENGTH: 27 base pairs
      (B) TYPE: nucleic acid
      (C) STRANDEDNESS: unknown
      (D) TOPOLOGY: unknown
   (ii) MOLECULE TYPE: cDNA
   (xi) SEQUENCE DESCRIPTION: SEQ ID NO:14:
      CCGGAGAAAC CATGTCCTGA CCCAGCA 27
(2) INFORMATION FOR SEQ ID NO:15:
   (i) SEQUENCE CHARACTERISTICS:
      (A) LENGTH: 51 base pairs
      (B) TYPE: nucleic acid
      (C) STRANDEDNESS: unknown
      (D) TOPOLOGY: unknown
   (ii) MOLECULE TYPE: cDNA
   (xi) SEQUENCE DESCRIPTION: SEQ ID NO:15:
      CCGGAGGCTA CCAACTCTAG CTCCAGCAGC TTCTCCTCTC CTAGCTCTGC A 51
(2) INFORMATION FOR SEQ ID NO:16:
   (i) SEQUENCE CHARACTERISTICS:
      (A) LENGTH: 43 base pairs
      (B) TYPE: nucleic acid
      (C) STRANDEDNESS: unknown
      (D) TOPOLOGY: unknown
   (ii) MOLECULE TYPE: cDNA
   (xi) SEQUENCE DESCRIPTION: SEQ ID NO:16:
      GAGCTAGGAG AGGAGAAGCT GCTGGAGCTA GAGTTGGTAG CCT 43